(19) **Europäisches Patentamt / European Patent Office / Office européen des brevets**

(11) **EP 3 240 902 B1**

(12) **FASCICULE DE BREVET EUROPEEN**

(45) Date de publication et mention de la délivrance du brevet:
**29.07.2020 Bulletin 2020/31**

(21) Numéro de dépôt: **15828828.2**

(22) Date de dépôt: **30.12.2015**

(51) Int Cl.:
*C12P 19/26* (2006.01)    *C08B 37/08* (2006.01)
*C12P 21/06* (2006.01)

(86) Numéro de dépôt international:
**PCT/FR2015/053781**

(87) Numéro de publication internationale:
**WO 2016/108033 (07.07.2016 Gazette 2016/27)**

(54) **CHITINE, HYDROLYSAT ET PROCÉDÉ DE PRODUCTION DE PRODUIT(S) D'INTÉRÊT À PARTIR D'INSECTES PAR HYDROLYSE ENZYMATIQUE**

CHITIN, HYDROLYSAT UND VERFAHREN ZUR HERSTELLUNG VON EINEM ODER MEHREREN GEWÜNSCHTEN PRODUKTEN AUS INSEKTEN DURCH ENZYMATISCHE HYDROLYSE

CHITIN, HYDROLYSATE AND METHOD FOR THE PRODUCTION OF ONE OR MORE DESIRED PRODUCTS FROM INSECTS BY MEANS OF ENZYMATIC HYDROLYSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.12.2014 FR 1463512**

(43) Date de publication de la demande:
**08.11.2017 Bulletin 2017/45**

(73) Titulaire: **Ynsect**
**91058 Évry-Courcouronnes Cedex (FR)**

(72) Inventeurs:
- **BEREZINA, Nathalie**
  **75005 Paris (FR)**
- **HUBERT, Antoine**
  **94140 Alfortville (FR)**
- **BERRO, Fabrice**
  **75001 Paris (FR)**
- **LEVON, Jean-Gabriel**
  **75011 Paris (FR)**
- **LE ROUX, Karine**
  **91490 Milly La Foret (FR)**
- **SOCOLSKY, Cecilia**
  **59800 Lille (FR)**
- **SANCHEZ, Lorena**
  **91260 Juvisy-sur-Orge (FR)**
- **LAURENT, Sophie**
  **44200 Nantes (FR)**

(74) Mandataire: **Santarelli**
**49, avenue des Champs-Elysées**
**75008 Paris (FR)**

(56) Documents cités:
CN-A- 1 297 691    CN-A- 101 775 085
CN-A- 103 725 742    CN-B- 101 144 097
FR-A1- 2 927 336    FR-A1- 2 975 706
US-A- 4 958 011

- DATABASE WPI Week 201301 2012 Thomson Scientific, London, GB; AN 2012-M24188 XP002747930, -& CN 102 558 387 A (QINGDAO ZHONGREN PHARMACY CO LTD) 11 juillet 2012 (2012-07-11)
- Nitar Nwe ET AL: "Chitin and Chitosan from Terrestrial Organisms" In: "Chitin, Chitosan, Oligosaccharides and Their Derivatives: Biological Activities and Applications", 1 octobre 2010 (2010-10-01), CRC Press, XP055221223, ISBN: 978-1-4398-1603-5 pages 3-10, alinéa [1.2.2]
- JUAN WANG ET AL: "Housefly larvae hydrolysate: orthogonal optimization of hydrolysis, antioxidant activity, amino acid composition and functional properties", BMC RESEARCH NOTES, BIOMED CENTRAL LTD, GB, vol. 6, no. 1, 17 mai 2013 (2013-05-17), page 197, XP021151554, ISSN: 1756-0500, DOI: 10.1186/1756-0500-6-197

- **DAI CHUNHUA ET AL: "Angiotensin I-converting enzyme (ACE) inhibitory peptide derived fromTenebrio molitor(L.) larva protein hydrolysate", EUROPEAN FOOD RESEARCH AND TECHNOLOGY, SPRINGER VERLAG, HEIDELBERG, DE, vol. 236, no. 4, 1 février 2013 (2013-02-01), pages 681-689, XP035311054, ISSN: 1438-2377, DOI: 10.1007/S00217-013-1923-Z [extrait le 2013-02-01]**

Remarques:
Le dossier contient des informations techniques présentées postérieurement au dépôt de la demande et ne figurant pas dans le présent fascicule.

**Description**

La présente invention concerne un procédé de production d'au moins un produit d'intérêt à partir d'insectes. Plus particulièrement, l'invention concerne un procédé de production de chitine et/ou de chitosan par hydrolyse enzymatique de cuticules d'insectes. Elle vise également une chitine spécifique et un hydrolysat.

Selon l'invention, par « chitine », on entend tout type de dérivé chitinique, c'est-à-dire de dérivé de polysaccharides comportant des unités N-acétyl-glucosamine et des unités D-glucosamines, en particulier les copolymères chitine-polypeptides (parfois désignés sous l'appellation « composite chitine-polypeptides »).

La chitine serait le deuxième polymère le plus synthétisé dans le monde vivant après la cellulose. En effet, la chitine est synthétisée par de nombreuses espèces du monde vivant: elle constitue en partie l'exosquelette des crustacés et des insectes et la paroi latérale qui entoure et protège les champignons. Plus particulièrement, chez les insectes, la chitine constitue ainsi 3 à 60% de leur exosquelette.

Par « chitosan », on entend selon la présente invention les produits de désacétylation de la chitine. La limite usuelle entre le chitosan et la chitine est déterminée par le degré d'acétylation : un composé ayant un degré d'acétylation inférieur à 50% est nommé chitosan, au-delà, un composé ayant un degré d'acétylation supérieur à 50% est nommé chitine.

Les applications de la chitine et/ou du chitosan sont nombreuses: cosmétique (composition cosmétique), médicale et pharmaceutique (composition pharmaceutique, traitement des brûlures, biomatériaux, pansements cornéens, fils chirurgicaux), diététique et alimentaire, technique (agent filtrant, texturant, floculant ou adsorbant notamment pour la filtration et dépollution de l'eau), etc. En effet, la chitine et /ou le chitosan sont des matériaux biocompatibles, biodégradables et non toxiques.

L'extraction traditionnelle de la chitine s'effectue par voie chimique à partir de crustacés, de céphalopodes, mais aussi, de manière plus exceptionnelle, de champignons. Cette voie emploie de grandes quantités de réactifs (tel que l'acide chlorhydrique, l'hydroxyde de sodium et des agents de blanchiment) qui ont pour effet de dénaturer la structure de la chitine telle qu'elle existe à l'état naturel, par exemple telle que présente dans la carapace des crustacés. En outre, la plupart des réactifs chimiques sont nocifs pour l'homme et l'environnement et génèrent d'importants volumes d'effluents à traiter. Enfin, la chitine et/ou le chitosan issu(s) de crustacés peuvent générer des réactions allergiques chez les personnes sensibles.

Une autre voie d'extraction de la chitine est la voie enzymatique. Cette voie est considérée comme plus douce, permettant ainsi de mieux préserver la chitine et/ou le chitosan. Toutefois, la chitine obtenue par cette voie est d'une couleur brunâtre, nécessitant des étapes de purification afin d'obtenir une poudre valorisable, c'est-à-dire de couleur blanche. Les procédés existants comportent donc généralement une ou plusieurs étape(s) visant à débarrasser la chitine de ses impuretés, telle qu'une étape de déminéralisation à l'acide réalisée préalablement à l'hydrolyse enzymatique et/ou une étape de blanchiment de la chitine avec un agent oxydant, réalisée de manière subséquente à l'hydrolyse enzymatique. Ces deux étapes de purification de la chitine ont malheureusement pour effet d'altérer la structure chimique de la chitine.

Le travail des inventeurs a permis de mettre en évidence qu'il était possible d'obtenir une chitine à la fois plus pure et de structure plus proche de la structure originelle de la chitine en effectuant une étape de traitement mécanique préalable à l'hydrolyse, à savoir une étape de pressage des cuticules d'insectes.

L'invention concerne donc un procédé de production d'au moins un produit d'intérêt à partir d'insectes, comportant les étapes suivantes :

> (i) le broyage des cuticules d'insectes,
> (ii) le pressage des cuticules d'insectes, puis,
> (iii) l'hydrolyse enzymatique des cuticules d'insectes par une enzyme protéolytique.

Par « produit d'intérêt », on entend plus particulièrement la chitine et/ou le chitosan et/ou un hydrolysat.

Par « hydrolysat », on désigne un produit qui comporte des protéines, des protéines hydrolysées, des peptides, des acides aminés et/ou d'autres composés dérivés d'une protéine, susceptibles d'être obtenus par hydrolyse enzymatique de protéines.

Le ou les produit(s) d'intérêt sont obtenus à partir d'insectes. Par « insectes », on entend des insectes à n'importe quel stade de développement, tel qu'un stade adulte, larvaire ou un stade de nymphe. De préférence, les insectes mis en oeuvre dans le procédé selon l'invention sont comestibles.

Plus particulièrement, les insectes peuvent être choisis parmi le groupe constitué par les coléoptères, les diptères, les lépidoptères, les isoptères, les orthoptères, les hyménoptères, les blattoptères, les hémyptères, les hétéroptères, les éphéméroptères et les mécoptères, de préférence, parmi les coléoptères, les diptères, les orthoptères et les lépidoptères.

Préférentiellement, les insectes sont choisis parmi le groupe constitué par *Tenebrio molitor, Hermetia illucens,*

*Galleria mellonella, Alphitobius diaperinus, Zophobas morio, Blattera fusca, Tribolium castaneum, Rhynchophorus ferrugineus, Musca domestica, Chrysomya megacephala, Locusta migratoria, Schistocerca gregaria, Acheta domesticus et Samia ricini.*

Plus préférentiellement, les insectes sont choisis parmi le groupe constitué par *Tenebrio molitor, Hermetia illucens, Galleria mellonella, Alphitobius diaperinus, Zophobas morio, Blattera fusca, Musca domestica, Chrysomya megacephala, Locusta migratoria, Schistocerca gregaria, Acheta domesticus* et *Samia ricini,* et plus préférentiellement encore, *T. molitor.*

Une ou plusieurs espèces d'insectes peuvent être utilisées dans le procédé selon l'invention, de préférence une seule espèce d'insecte. Si plusieurs espèces sont utilisées, on choisira avantageusement deux espèces proches telles que par exemple, *Hermetia illucens* et *Musca domestica.*

Les insectes sont de préférence élevés et non prélevés dans la nature. Par exemple, les insectes sont élevés dans une ferme d'insectes. L'élevage des insectes dans une ferme spécifique permet non seulement de contrôler et d'éliminer les risques associés aux maladies véhiculées par des insectes, mais également de limiter les risques associés à la toxicité des produits alimentaires dérivés des insectes due par exemple à la présence d'insecticides. En outre, l'élevage permet de contrôler la qualité de l'approvisionnement en insectes et de limiter les coûts d'approvisionnement.

Par « cuticules d'insectes », on vise non seulement les cuticules une fois celles-ci séparées des insectes, mais également les cuticules incluant tout ou partie des autres constituants de l'insecte, y compris l'insecte dans son intégralité. En effet, il est possible d'appliquer le procédé selon l'invention à l'insecte complet, tels que des insectes broyés, ou bien à seulement une partie des insectes comportant les cuticules, par exemple les exuvies et/ou les mues d'insectes, séparées naturellement et collectées par un procédé adéquat.

La cuticule est la couche externe (ou exosquelette) sécrétée par l'épiderme des insectes. Elle est en général formée de trois couches :

- l'épicuticule, qui est la couche la plus fine et la plus externe de la cuticule (inférieure à 4 $\mu$m) ; cette couche est imperméable à l'eau et comporte une couche de cire imperméabilisante, ainsi que des protéines et de la chitine, en quantité moindre ;
- l'exocuticule, qui est la couche intermédiaire de la cuticule ; elle est composée essentiellement de protéines durcies, les tannées, qui sont responsables de la rigidité de la cuticule, de chitine et éventuellement de mélanine ; et
- l'endocuticule, qui est une couche fine, flexible, constituée d'un mélange de protéines et de chitine.

Le pressage des cuticules d'insectes a pour objectif principal d'éliminer un jus de presse riche en matière grasse et/ou d'enrichir le gâteau de presse en substrat pour l'hydrolyse.

Dans le procédé selon l'invention, le pressage des cuticules d'insectes permet d'obtenir un gâteau de presse comportant une teneur en huile (ou lipides) inférieure ou égale à 20%, préférentiellement, inférieure ou égale à 15%, plus préférentiellement, inférieure ou égale à 12%, encore plus préférentiellement, inférieure ou égale à 10%.

Dans la présente demande, les gammes de valeurs s'entendent bornes incluses. De même, lorsqu' « environ » ou « de l'ordre de » précède un nombre, cela équivaut à plus ou moins 10% de la valeur de ce nombre.

Par ailleurs, afin d'enrichir le gâteau de presse en substrat pour l'hydrolyse, le pressage des cuticules d'insectes permet d'obtenir un gâteau de presse présentant une teneur en matière sèche comprise entre 30% et 60%, préférentiellement, comprise entre 40% et 55%, et plus préférentiellement comprise entre 45% et 50%.

Tout système de presse peut être utilisé pour réaliser le pressage des cuticules d'insectes, tel que par exemple, une presse mono-vis ou bi-vis (presse bi-vis de type Angel), un filtre-presse (filtre-presse de type Choquenet), une presse à plateaux, etc. Ces systèmes sont bien connus de l'homme du métier qui est à même de déterminer les conditions de pressage afin d'obtenir les teneurs en huile et/ou en eau mentionnées ci-avant.

Dans le procédé selon l'invention, le pressage des cuticules d'insectes est suivi d'une hydrolyse enzymatique.

De préférence, l'hydrolyse enzymatique est effectuée par au moins une enzyme protéolytique, de préférence une protéase. Dans la présente demande, les noms ou suffixes « peptidase » et « protéase » sont utilisés indifféremment pour désigner une enzyme lysant une liaison peptidique des protéines. Avantageusement, celle-ci est effectuée pendant une durée de 4 à 8h, préférentiellement, pendant 4 à 5h, à une température de 40 à 60°C, préférentiellement, 45 à 55°C et à un pH compris entre 6 et 8, préférentiellement entre 6,5 et 7,5.

L'hydrolyse enzymatique peut être réalisée avec une seule protéase ou alternativement avec un mélange d'enzymes contenant au moins une protéase, plus préférentiellement un mélange d'enzymes contenant plusieurs protéases, tel qu'un mélange contenant une endoprotéase et une exoprotéase, ou une protéase et une polysaccharase.

De préférence, la protéase est choisie parmi le groupe constitué par les aminopeptidases, les métallocarboxypeptidases, les endopeptidases sérine, les endopeptidases cystéine, les endopeptidases aspartiques, les métalloendopeptidases.

Avantageusement, les enzymes peuvent être choisies parmi les suivantes :

| Enzyme(s) | Classe | Numéro EC | Fournisseur | Ville | Pays |
|---|---|---|---|---|---|
| Flavourzyme | Aminopeptidases | EC 3.4.11.1 | Novozyme | Bagsvaerd | Danemark |
| Fungal protease 500 | | EC 3.4.11.1 | Bio-Cat | Troy | Etats-Unis |
| Kojizyme | | EC 3.4.11.1 | Novozyme | Bagsvaerd | Danemark |
| Protex P | Endopeptidases sérine | EC 3.4.21 | Genencor International B.V. | Leiden | Pays-Bas |
| Chymotrypsine | | EC 3.4.21.1 | Novozyme | Bagsvaerd | Danemark |
| Protamex | | EC 3.4.21 | Novozyme | Bagsvaerd | Danemark |
| Elastase | | EC 3.4.21.14 | Novozyme | Bagsvaerd | Danemark |
| Trypsine | | EC 3.4.21.36 | Novozyme | Bagsvaerd | Danemark |
| Alcalase | | EC 3.4.21.4 | Novozyme | Bagsvaerd | Danemark |
| Papaïne | Endopeptidases cystéine | EC 3.4.22.2 | Bio-Cat | Troy | Etats-Unis |
| Bromelaine (ananase) | | EC 3.4.22.32 | Bio-Cat | Troy | Etats-Unis |
| Prolyve NP | Endopeptidases aspartique | EC 3.4.23 | Lyven | Colombelles | France |
| Pepsine | | EC 3.4.23.1 | Sigma Aldrich | Saint-Quentin-Fallavier | France |
| Neutral protéase | Métallo-endo-peptidase | EC 3.4.24.28 | Bio-Cat | Troy | Etats-Unis |
| Protex 50FP | Endo-peptidase | EC 3.4.21 | Genencor International B.V. | Leiden | Pays-Bas |
| Pancrealyve | Exo & endo peptidase (cocktail protéases + amylases) | n.a.* | Lyven | Colombelles | France |
| Izyme BA | Protéase aspartique | EC 3.4.23 | Novozyme | Bagsvaerd | Danemark |
| Sumizyme | Cocktail enzymatique | n.a.* | Takabio - Shin Nihon | Aichi | Japon |
| Neutrase | Endoprotéase base de Zn de $\beta$ amyloliquefaciens | EC 3.4.24 | Novozyme | Bagsvaerd | Danemark |
| Novozyme 37071 | Protéase | n.a.* | Novozyme | Bagsvaerd | Danemark |
| *n.a. : non applicable | | | | | |

Avantageusement, l'enzyme mise en œuvre lors de l'hydrolyse est une endopeptidases aspartique, telle que la Prolyve NP. Ce type d'enzyme permet d'obtenir de très bons résultats en terme de pureté de la chitine obtenue, en particulier lorsque ce type d'enzyme est appliqué à l'hydrolyse d'un gâteau de presse obtenu à partir de coléoptères et plus particulièrement de *T. molitor.*

L'enzyme ou le mélange d'enzymes est introduit(e) à une quantité allant de 0,2 à 10 % en poids de matière sèche estimé, préférentiellement de 0,4 à 8% en poids et plus préférentiellement de 0,5 à 2%. Par « poids de matière sèche estimé », on vise plus particulièrement le poids de matière sèche d'insectes ou de partie(s) d'insectes, tel qu'on peut l'estimer lors de l'entrée en étape d'hydrolyse enzymatique.

En termes d'activité enzymatique, la quantité d'enzyme ou de mélange d'enzymes introduite est équivalente à une activité comprise entre 2000 et 5000 SAPU (« *Spectrophotometric Acid Protease Unit* », décrit dans l'Exemple 5 ci-après), de préférence comprise entre 3000 et 4000 SAPU, pour 100 g en poids humide, avec une humidité de 30 à 70%, de substrat à transformer, c'est-à-dire de matière d'insectes ou de partie(s) d'insectes hydratée.

Avantageusement, l'étape d'hydrolyse enzymatique s'effectue en présence d'eau, telle que de l'eau douce. La quantité d'eau mise en oeuvre lors de l'hydrolyse enzymatique est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1. On notera que ce ratio correspond également au ratio du poids d'eau sur le poids d'insecte, la masse volumique de l'eau étant de 1,0 g/mL dans les conditions normales de température et de pression.

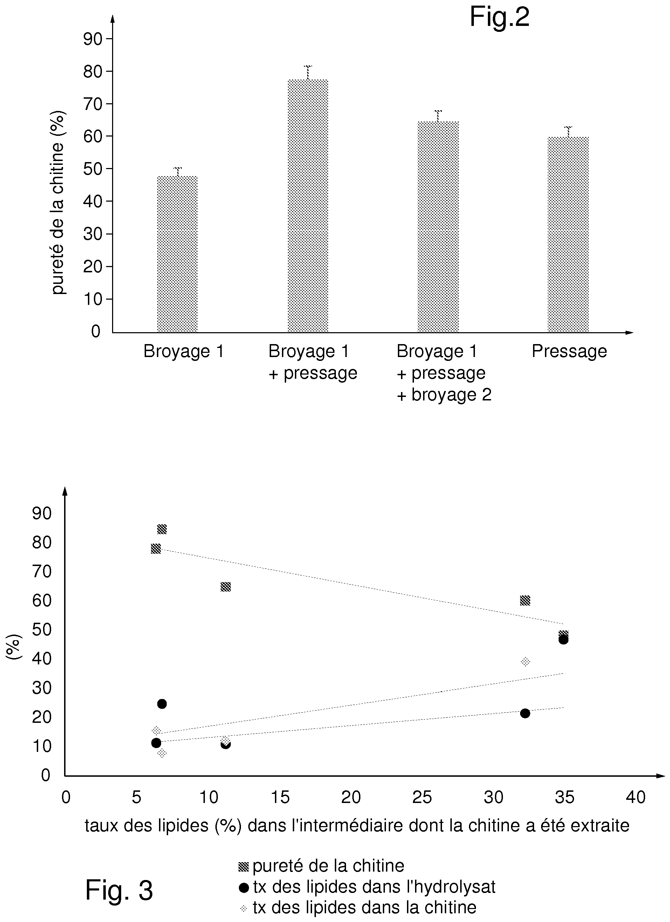

Le procédé selon l'invention permet l'obtention d'une chitine ayant un degré de pureté (ou pureté gravimétrique) compris entre 40 et 90%, préférentiellement entre 50 et 90%, plus préférentiellement compris entre 60 et 85%, et encore plus préférentiellement de l'ordre de 80% (voir l'Exemple 2 et la Figure 2).

De plus, le procédé selon l'invention permet l'obtention d'un hydrolysat présentant un certain nombre de propriétés avantageuses, en particulier en terme de digestibilité, de teneur en lipides et/ou protéines, de taille de protéine ou de composition en acides aminés.

Le procédé selon l'invention comporte une étape de broyage préalable à l'étape de pressage.

Cette étape de broyage a pour objectif de réduire les cuticules et/ou les insectes en particules afin de faciliter l'accès des enzymes au substrat lors de l'hydrolyse enzymatique. Cette étape permet également, lorsqu'elle est suivie d'une étape de pressage, de faciliter l'élimination du jus de presse et l'isolation de la matière solide.

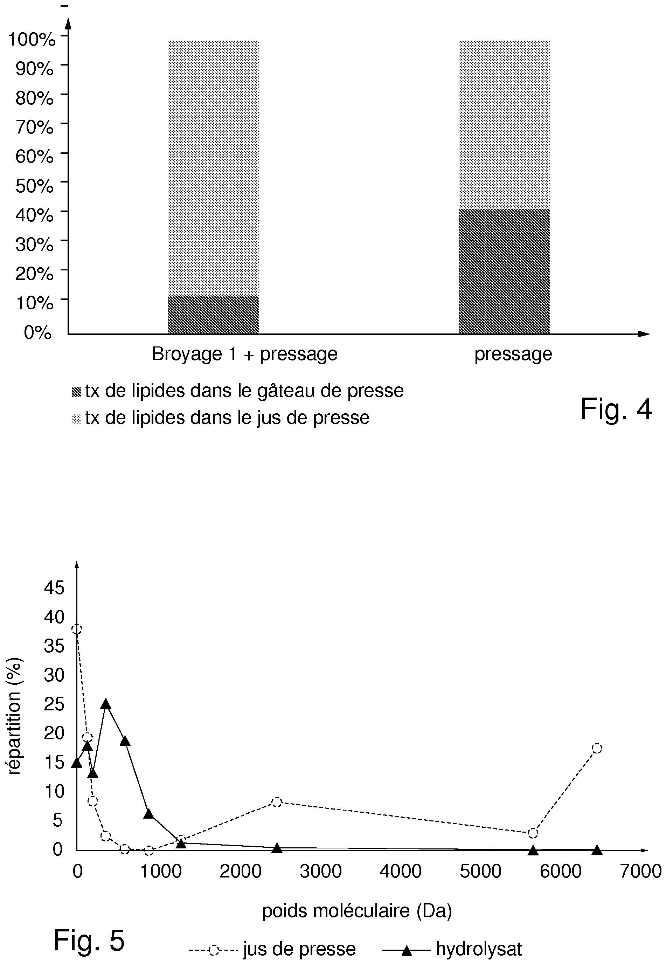

Cette étape de broyage permet également d'améliorer la répartition des lipides issus de l'insecte entre le gâteau de presse et le jus de presse issus du pressage. En effet, comme cela est illustré en Figure 4, le gâteau de presse issu d'un procédé comportant à la fois broyage et pressage présente une teneur en lipides réduite en comparaison avec un gâteau de presse d'un procédé comportant uniquement un pressage.

Plus particulièrement, dans le procédé selon l'invention, le broyage et le pressage des cuticules d'insectes permet d'obtenir un gâteau de presse comportant une teneur en lipides (ou huile) inférieure ou égale à 15%, préférentiellement, inférieure ou égale à 12%, encore plus préférentiellement, inférieure ou égale à 10%.

Le broyage peut avantageusement être effectué avec un broyeur mixeur, tel qu'un broyeur mixeur à couteaux.

De préférence, à l'issue du broyage, la taille des particules d'insectes est inférieure à 1 cm (plus grande taille de particule observable à l'aide d'un microscope), de préférence inférieure à 0,5 cm, plus préférentiellement encore, une taille comprise entre 300 $\mu$m et 0,5 cm, préférentiellement 500 $\mu$m et 0,5 cm et encore plus préférentiellement entre 500 $\mu$m et 1 mm.

Afin de faciliter le broyage, une quantité d'eau peut être ajoutée. Cette quantité d'eau est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Avantageusement, le procédé selon l'invention comporte également une étape d'abattage des insectes préalable à l'étape de pressage et/ou de broyage.

Cette étape d'abattage peut s'effectuer par des méthodes classiques d'élevage d'animaux à sang froid et/ou de petite taille (crustacés, poissons, escargots, etc.), tels que le froid (congélation), le chaud (ébouillantage), la privation d'oxygène, etc. Avantageusement, l'étape d'abattage des insectes est effectuée par ébouillantage. L'ébouillantage permet non seulement d'abattre les insectes, mais également d'abaisser la charge microbienne (réduction du risque d'altération et sanitaire) et d'inactiver les enzymes internes des insectes pouvant déclencher une autolyse, et ainsi un brunissement rapide de ceux-ci. Cet ébouillantage est réalisé de manière à provoquer la mort le plus rapidement possible, dans le respect du bien-être animal, et selon les préconisations scientifiques.

Alternativement, l'abattage peut être effectué par blanchiment. Le blanchiment présente les mêmes avantages cités ci-avant que l'ébouillantage.

Avantageusement, les insectes sont abattus, par exemple par ébouillantage ou blanchiment, puis broyés avant d'être pressés.

De préférence, l'étape d'ébouillantage est réalisée dans l'eau, telle que de l'eau douce, à une température de 95 à 105°C, préférentiellement de l'ordre de 100°C et pendant une durée de 2 à 20 min, préférentiellement, 5 à 15 min.

La quantité d'eau introduite à cette étape d'ébouillantage est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Alternativement, l'étape de blanchiment est réalisée à la vapeur d'eau et/ou à l'eau à une température comprise entre 80°C et 130°C, de préférence entre 90°C et 120°C.

Le procédé selon l'invention peut comporter, en outre, une étape de traitement des cuticules des insectes par un agent oxydant préalablement à l'hydrolyse enzymatique.

De préférence, dans le procédé selon l'invention, l'agent oxydant mis en oeuvre lors du traitement des cuticules est choisi parmi le groupe constitué par le peroxyde d'hydrogène, le permanganate de potassium, l'ozone et l'hypochlorite de sodium, plus préférentiellement encore, le peroxyde d'hydrogène.

Avantageusement, lorsque l'agent oxydant est le peroxyde d'hydrogène, la quantité introduite de cet agent pour le traitement des cuticules d'insectes est telle que la teneur en peroxyde d'hydrogène est comprise entre 1 et 33% en poids sur le poids total d'insectes, préférentiellement, entre 2 et 12% en poids sur le poids total d'insectes, préférentiellement de l'ordre de 6% en poids.

Préférentiellement, le traitement de cuticules d'insectes par l'agent oxydant s'effectue en présence d'eau, telle que de l'eau douce. Avantageusement, la quantité d'eau mise en oeuvre lors du traitement des cuticules est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Le traitement des cuticules d'insectes avec l'agent oxydant peut être effectué pendant l'une ou plusieurs des étapes ci-après :

- concomitamment avec l'ébouillantage et/ou après l'étape d'ébouillantage, plus préférentiellement concomitamment avec l'ébouillantage. Alternativement, concomitamment avec le blanchiment et/ou après l'étape de blanchiment, plus préférentiellement concomitamment avec le blanchiment. Plus particulièrement, lorsque le traitement des cuticules d'insectes est effectué pendant l'ébouillantage ou le blanchiment, l'agent oxydant peut être avantageusement ajouté à l'eau utilisée pour ébouillanter les insectes.
- avant, concomitamment et/ou après le broyage. Plus particulièrement, lorsque le traitement des cuticules d'insectes est effectué pendant le broyage, l'agent oxydant peut être avantageusement ajouté à l'eau utilisée pour le broyage.
- avant et/ou concomitamment avec le pressage.
- lors d'une étape spécifique de traitement des cuticules d'insectes.

Avantageusement, l'hydrolyse enzymatique peut être suivie d'une étape d'inactivation thermique visant à inactiver l'enzyme ou le mélange d'enzymes mis en oeuvre lors de l'hydrolyse enzymatique.

A l'issue d'un procédé selon l'invention, la chitine peut être récupérée par pressage ou centrifugation du milieu de réaction de l'hydrolyse enzymatique. A ce stade, un co-produit de la chitine est également récupéré, à savoir un hydrolysat.

Un mode de réalisation préférentiel d'un procédé selon l'invention est plus détaillé ci-après.

En particulier, ce mode de réalisation préférentiel décrit diverses étapes avantageuses pour un procédé selon l'invention, telles que des étapes de purification douce de la chitine : un second pressage, des lavages, filtration et séchage éventuels.

Enfin, la chitine étant généralement commercialisée sous forme de poudre, un second broyage peut également être réalisé. Celui-ci peut également être effectué afin de favoriser la réaction de désacétylation, qui permet de préparer du chitosan à partir de chitine. Les conditions de la réaction de désacétylation sont plus amplement décrites à l'étape 10 du mode de réalisation préférentiel détaillé ci-après.

Un procédé particulièrement avantageux de production de chitine à partir de cuticules d'insectes, comporte les étapes suivantes :

  a) l'abattage des insectes,
  b) le broyage des insectes,
  c) le pressage des insectes,
  d) l'hydrolyse enzymatique des cuticules d'insectes par une enzyme protéolytique,
  e) la récupération de la chitine,

les cuticules d'insectes pouvant optionnellement être traitées avec un agent oxydant avant l'étape d).

Les modes de réalisation préférés des diverses étapes a) à e) ainsi que le traitement avec l'agent oxydant sont tels qu'indiqués ci-avant ou dans l'étape correspondante dans le mode de réalisation préférentiel ci-après.

L'invention concerne également une chitine, telle qu'une chitine susceptible d'être obtenue par un procédé selon l'invention. Grâce aux conditions douces employées dans le procédé selon l'invention, cette chitine possède une structure proche de la chitine telle que présente à l'état naturel dans la cuticule de l'insecte tout en ayant un haut degré de pureté, tel qu'un degré de pureté compris entre 40 et 90%, préférentiellement entre 50 et 90%, plus préférentiellement compris entre 60 et 85%, et encore plus préférentiellement de l'ordre de 80%.

La chitine susceptible d'être obtenue par un procédé selon l'invention présente au moins l'une quelconque des propriétés avantageuses suivantes :

- un taux de cendre inférieur à 4%, préférentiellement inférieur à 3,5%, plus préférentiellement inférieur ou égal à 2,5% (en particulier lorsque la chitine est préparée à partir d'insectes végétariens), plus préférentiellement inférieur à 2%, et encore plus préférentiellement inférieur ou égal à 1,5% (en particulier lorsque la chitine est préparée à partir de *T. molitor*), et encore plus préférentiellement inférieur à 1% en poids sur le poids total de matière sèche,
- degré de pureté (ou pureté gravimétrique) compris entre 40 et 90%, préférentiellement entre 50 et 90%, plus préférentiellement compris entre 60 et 85%, et encore plus préférentiellement de l'ordre de 80%,
- une abondance fonctionnelle en surface présentant un ratio (C-O)/(C-H) compris entre 0,30 et 0,56, préférentiellement, entre 0,31 et 0,53,
- Taux de lipides ≤14% en poids total sur le poids total de matière sèche,
- Taux de lipides ≤5% lorsque la chitine est préparée à partir d'insectes non volants,
- Total des acides aminés ≤45%,
- Total des acides aminés ≤16% lorsque la chitine est préparée à partir d'insectes volants,
- Taux d'abondance relative de au moins 3 quelconque acides aminés parmi ALA, GLY, LEU, PRO, SER, TYR, VAL ≤10%,
- Taux d'abondance relative de LEU, PRO, VAL ≤10% lorsque la chitine est préparée à partir de *T. molitor,*
- Taux d'abondance relative de ALA ≤12% lorsque la chitine est préparée à partir de *T. molitor,*
- Pureté colorimétrique ≥40%,
- Pureté colorimétrique ≥50% lorsque la chitine est préparée avec l'enzyme prolyve NP lors de l'hydrolyse enzymatique,
- Pureté par différence ≥45%, préférentiellement ≥49%,
- Pureté par différence ≥52% lorsque la chitine est préparée à partir de *T. molitor,*
- Pureté par différence ≥70% lorsque la chitine est préparée à partir d'insectes volants,
- Degré d'acétylation ≥70% et degré de cristallinité ≤0,61,
- Degré d'acétylation ≥70% et degré de cristallinité ≥0,42.

La chitine selon l'invention comporte un taux d'acides aminés inférieur ou égal à 45% en poids sur le poids total de matière sèche, un taux de cendres inférieur ou égal à 2,5% en poids sur le poids total de matière sèche, une pureté par différence supérieure ou égale à 45%, un degré d'acétylation supérieur ou égal à 70% et un degré de cristallinité supérieur ou égal à 0,42.

De préférence, la chitine présente toutes les propriétés ci-avant.

Toutes les unités et méthodes de mesure des caractéristiques indiquées ci-avant sont décrites dans les Exemples et, plus particulièrement, dans l'Exemple 5.

Un procédé particulièrement avantageux de production d'un hydrolysat à partir d'insectes comporte les étapes suivantes :

a) l'abattage des insectes,
b) le broyage des insectes,
c) le pressage des insectes,
d) l'hydrolyse enzymatique des cuticules d'insectes par une enzyme protéolytique,
e) la récupération de l'hydrolysat,

les cuticules d'insectes pouvant optionnellement être traitées avec un agent oxydant avant l'étape d).

Les modes de réalisation préférés des diverses étapes a) à e) ainsi que le traitement avec l'agent oxydant sont tels qu'indiqués ci-avant ou dans l'étape correspondante dans le mode de réalisation préférentiel ci-après.

L'invention concerne également un hydrolysat, tel qu'un hydrolysat susceptible d'être obtenu par un procédé selon l'invention.

L'hydrolysat susceptible d'être obtenu par un procédé selon l'invention présente au moins l'une quelconque des propriétés avantageuses suivantes :

- un taux de cendre inférieur à 4%, préférentiellement inférieur à 3% en poids (en particulier lorsque l'hydrolysat est préparé à partir d'insectes végétariens) sur le poids total de matière sèche,
- une excellente digestibilité, en particulier une digestibilité pepsique, supérieure à 95%, préférentiellement supérieure ou égale à 96% et plus préférentiellement supérieure ou égale à 98%, et plus particulièrement supérieure ou égale à 99%, la digestibilité pepsique ayant été mesurée selon une méthode conforme à la directive 72/188/EEC ;

- une teneur en protéines/peptides supérieure ou égale à 70%, préférentiellement supérieure ou égale à 71%, et plus préférentiellement supérieure ou égale à 75%, et plus préférentiellement supérieure ou égale à 80% (en particulier, lorsque l'hydrolysat est préparé à partir d'insectes non volant) ;

- une teneur en protéines hydrosolubles présentant une taille supérieure à 12400 g/mol, inférieure à 20%, de préférence, inférieure à 18% en poids sur le poids total de protéines hydrosolubles ;
- une teneur en lipides inférieure à 14%, préférentiellement inférieure à 10%, plus préférentiellement inférieure ou égale à 5% (en particulier lorsque l'hydrolysat est préparé à partir d'insectes non volants), plus préférentiellement inférieure ou égale à 2%, et encore plus préférentiellement inférieure ou égale à 1% ;
- une composition dans laquelle les acides aminés les plus abondants sont la proline, l'alanine, la leucine, l'acide aspartique, la glutamine et/ou la valine. Par « plus abondant », on entend une quantité relative de l'acide aminé dans la composition supérieure à 5%, préférentiellement supérieure à 7% en poids sur le poids total d'acides aminés. La forte teneur en proline s'avère particulièrement intéressante ;
- Taux d'abondance relative de au moins 5 quelconques acides aminés choisis parmi ASP, GLU, ALA, GLY, LEU, PRO, TYR, VAL, LYS ≥6% ;
- Taux d'abondance relative de au moins 3 quelconques acides aminés choisis parmi ASP, GLU, ALA, LEU, PRO, TYR, VAL ≥8%.

Plus particulièrement, par « protéines hydrosolubles », on entend, parmi les protéines (ou protéines brutes), celles qui sont solubles dans une solution aqueuse dont le pH est compris entre 6 et 8, avantageusement entre 7,2 et 7,6.
De préférence, la solution aqueuse est une solution tampon dont le pH est compris entre 6 et 8, avantageusement entre 7,2 et 7,6.
Préférentiellement, la solution tampon est une solution phosphate NaCl, dont le pH est égale à 7,4 $\pm$ 0,2.
L'hydrolysat selon l'invention comporte au moins 40% de protéines en poids sur le poids total de matière sèche, un taux de cendre inférieur à 3% en poids sur le poids total de matière sèche, et un taux de protéines hydrosolubles ayant une taille supérieure à 12400 g/mol inférieur à 50%, préférentiellement inférieure à 43%.
Par « insecte végétarien », on vise un insecte qui n'a pas de protéines animales dans son régime alimentaire habituel. A titre d'exemple d'insecte végétarien, on peut citer les coléoptères, les lépidoptères ou les orthoptères.
Par « insecte volant », on vise un insecte qui est capable de voler à l'âge adulte, contrairement à un insecte dit « non volant ». A titre d'exemple d'insecte volant, on peut citer les lépidoptères ou les diptères. A titre d'exemple d'insecte non volant, on peut citer les coléoptères ou les orthoptères.
Toutes les unités et méthodes de mesure des caractéristiques indiquées ci-avant sont décrites dans les Exemples et, plus particulièrement, dans l'Exemple 5.
De préférence, l'hydrolysat présente toutes les propriétés ci-avant.
On notera en particulier que l'hydrolysat peut être distingué de tout autre type d'hydrolysat par sa teneur en glucosamine, et/ou l'un de ses dérivés (préférentiellement N-acétyl-glucosamine), plus particulièrement une teneur supérieure ou également à 0,01%, préférentiellement supérieure ou égale à 0,08% en poids sur le poids total de matière sèche de l'hydrolysat.
Dans la présente demande, toute référence à un règlement ou à une directive concerne ledit règlement ou ladite directive telle qu'en vigueur à la date de dépôt de la présente demande.
L'hydrolysat peut avantageusement être complété avec des additifs pour équilibrer son profil nutritionnel afin d'être adapté à différents types d'animaux.
L'hydrolysat peut avantageusement être concentré puis séché pour obtenir un hydrolysat séché. Alternativement, l'hydrolysat peut être sous forme liquide. Ces hydrolysats peuvent être utilisés comme un aliment ou un ingrédient alimentaire en particulier pour des animaux, ou, alternativement, ils peuvent être traités, par exemple, pour isoler des acides aminés.
Un procédé particulièrement avantageux de production de chitosan à partir de cuticules d'insectes, comporte les étapes suivantes :

a) l'abattage des insectes,
b) le broyage des insectes,
c) le pressage des insectes,
d) l'hydrolyse enzymatique des cuticules d'insectes par une protéase,
e) la récupération de la chitine,
f) la désacétylation de la chitine récupérée,
g) la récupération du chitosan,

les cuticules d'insectes pouvant optionnellement être traitées avec un agent oxydant avant l'étape d).

Les modes de réalisation préférés des diverses étapes a) à g) ainsi que le traitement avec l'agent oxydant sont tels qu'indiqués ci-avant ou dans l'étape correspondante dans le mode de réalisation préférentiel ci-après.

L'invention concerne également un chitosan susceptible d'être obtenu par un procédé selon l'invention.

La chitine et/ou le chitosan susceptible(s) d'être obtenu(s) par un procédé selon l'invention peu(ven)t avantageusement être utilisé(s) dans diverses applications :

- dans des compositions cosmétiques, pharmaceutiques, nutraceutiques ou diététiques,
- comme biomatériaux pour traiter des brûlures en tant que seconde peau, pour réaliser des pansements cornéens ou des fils chirurgicaux,
- comme agent filtrant, texturant, floculant et/ou adsorbant notamment pour la filtration et dépollution de l'eau.

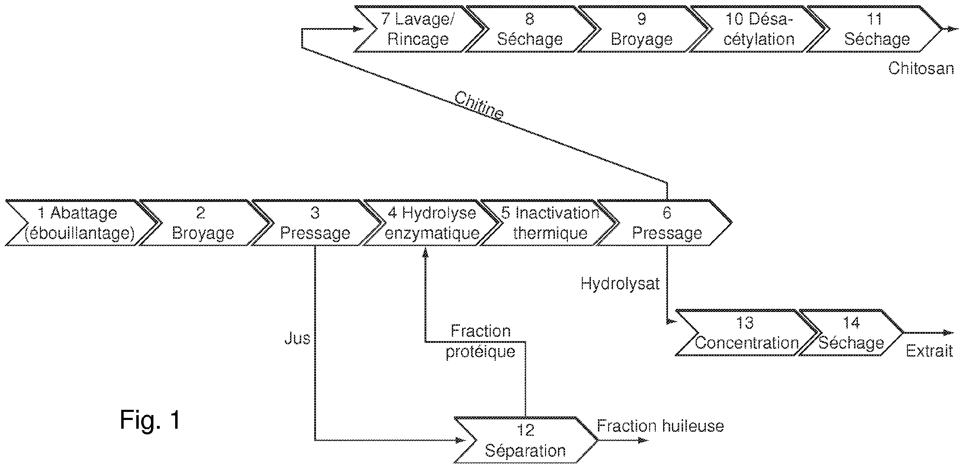

Selon un mode de réalisation préférentiel de l'invention, le procédé comporte les étapes suivantes, décrites schématiquement en Figure 1. On notera que certaines étapes sont indiquées comme facultatives dans ce mode de réalisation préférentiel.

#### • Etape 1 : abattage des insectes

Cette étape 1 d'abattage permet d'abattre les insectes tout en abaissant la charge microbienne (réduction du risque d'altération et sanitaire) et en inactivant les enzymes internes des insectes pouvant déclencher une autolyse, et ainsi un brunissement rapide de ceux-ci.

L'abattage peut s'effectuer par ébouillantage.

Les insectes, de préférence des larves, sont ainsi ébouillantés à l'eau pendant 2 à 20 min, préférentiellement, 5 à 15 min. De préférence, l'eau est à température comprise entre 95 à 105°C, préférentiellement 100°C.

La quantité d'eau introduite à cette étape 1 d'ébouillantage est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Alternativement, l'abattage peut s'effectuer par blanchiment. De préférence, les insectes sont blanchis à la vapeur (buses ou lit de vapeur) à une température comprise entre 80 et 130°C, de préférence entre 90 et 120°C, plus préférentiellement entre 95 et 105°C, encore plus préférentiellement 98°C ou bien à l'eau à une température comprise entre 95 et 105°C, préférentiellement 100°C (par buses d'aspersion) ou en mode mixte (eau + vapeur) à une température comprise entre 80 et 130°C, de préférence entre 90 et 120°C, plus préférentiellement entre 95 et 105°C. Le temps de séjour dans la chambre de blanchiment est compris entre 1 et 15 minutes, préférentiellement entre 3 et 7 min.

Lors de cette étape, il est également possible de traiter les cuticules d'insectes en utilisant une eau d'ébouillantage ou de blanchiment comportant l'agent oxydant selon les modalités indiquées dans l'étape intermédiaire ci-après.

#### • Etape intermédiaire (facultative) : traitement des cuticules par l'agent oxydant

Il est possible d'introduire dans le procédé une étape spécifique de traitement des cuticules par l'agent oxydant. Avantageusement, cette étape intermédiaire de traitement des cuticules s'effectue entre l'étape 1 d'abattage et l'étape 2 de broyage.

Cette étape intermédiaire s'effectue de préférence avec un agent oxydant choisi parmi le groupe constitué par le peroxyde d'hydrogène ($H_2O_2$), le permanganate de potassium ($KMnO_4$), l'ozone ($O_3$) et l'hypochlorite de sodium (NaClO), plus préférentiellement, le peroxyde d'hydrogène.

Selon un premier mode de réalisation, à l'issue de l'étape 1, lorsque celle-ci est faite par ébouillantage, on introduit directement dans la cuve d'ébouillantage l'agent oxydant, après éventuel refroidissement de l'eau d'ébouillantage à une température de l'ordre de 40 à 60°C, préférentiellement de l'ordre de 50°C.

Le peroxyde d'hydrogène tel que commercialisé est usuellement sous la forme d'une solution aqueuse, par exemple une solution à 30% en poids sur le poids total d'eau.

La quantité introduite de peroxyde d'hydrogène pour le traitement est telle que la teneur en peroxyde d'hydrogène est comprise entre 1 à 33% en poids sur le poids total d'insectes, préférentiellement, 2 à 12% en poids sur le poids total d'insectes, préférentiellement de l'ordre de 6% en poids.

Selon un second mode de réalisation, les insectes sont retirés de la cuve d'ébouillantage, tamisés et réintroduits dans une cuve.

Le peroxyde d'hydrogène est alors introduit dans la cuve sous la forme d'une solution aqueuse diluée, la teneur en peroxyde d'hydrogène étant alors comprise entre 1 et 33% en poids sur le poids d'eau, préférentiellement, 2 à 12% en poids sur le poids d'eau, préférentiellement de l'ordre de 6% en poids.

**• Etape 2 : broyage**

Les insectes sont retirés de la cuve d'ébouillantage ou de traitement ou de la chambre de blanchiment, ils sont ensuite tamisés, et placés dans un broyeur, tel qu'un broyeur mixeur à couteaux, permettant de réduire les insectes en particules.

Afin de faciliter le broyage, une quantité d'eau peut être ajoutée. Cette quantité d'eau est similaire à celle introduite lors de l'étape 1 d'ébouillantage : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1. Il est également possible de garder l'eau d'ébouillantage et/ou l'eau de l'étape intermédiaire pour effectuer cette étape. Cette eau est susceptible de contenir l'oxydant. Dans ce cas, le traitement des cuticules peut avoir lieu durant l'étape 1 d'ébouillantage et l'étape 2 de broyage ou pendant l'étape intermédiaire de traitement des cuticules et pendant l'étape de broyage.

De préférence, à l'issue du broyage, la taille des particules d'insectes est inférieure à 1 cm (plus grande taille de particule observable à l'aide d'un microscope), de préférence inférieure à 0,5 cm. Préférentiellement, la taille des particules est comprise entre 500 μm et 0,5 cm et encore plus préférentiellement entre 500 μm et 1 mm.

Il n'est pas nécessaire de réduire excessivement la taille des particules, par exemple à une taille inférieure à 250 μm.

Cette étape 2 de broyage favorise l'accès des enzymes à leur substrat.

Lors de cette étape, il est possible d'introduire dans le broyeur l'agent oxydant afin de traiter les cuticules selon les modalités indiquées dans l'étape intermédiaire ci-avant.

Lorsque le traitement des cuticules n'est pas effectué de manière concomitante avec le broyage, il est possible d'ajouter lors de cette étape, des additifs antioxydants communément employés pour la conservation et la stabilité du produit.

**• Etape 3 : pressage des cuticules d'insectes**

La pâte humide issue de l'étape 2 de broyage est ensuite placée dans une presse selon un mode opératoire qui permet de presser et séparer un jus comportant à la fois une fraction huileuse et une fraction protéique.

De préférence, l'étape de pressage permet d'obtenir un gâteau de presse comportant une teneur en huile inférieure ou égale à 20%, préférentiellement, inférieure ou égale à 15%, plus préférentiellement, inférieure ou égale à 12%, encore plus préférentiellement, inférieure ou égale à 10%.

De même, l'étape de pressage permet d'obtenir un gâteau de presse présentant une teneur en matière sèche comprise entre 30% et 60%, préférentiellement, comprise entre 40% et 55%, et plus préférentiellement comprise entre 45% et 50%.

Tout système de presse peut être utilisé pour réaliser l'étape de pressage, tel que par exemple, une presse mono-vis ou bi-vis (presse bi-vis de type Angel), un filtre-presse (filtre-presse de type Choquenet), une presse à plateaux, etc. Ces systèmes sont bien connus de l'homme du métier qui est à même de déterminer les conditions de pressage afin d'obtenir les teneurs en huile et/ou en eau mentionnées ci-avant.

Si la pâte humide issue de l'étape 2 de broyage a été obtenue avec une eau comportant l'oxydant, il peut être avantageux d'éliminer au moins une partie de cet oxydant avant l'étape 3 de pressage.

Cette étape 3 de pressage peut éventuellement être réalisée avant l'étape 2 de broyage à partir des insectes ébouillantés. Toutefois, il est avantageux d'effectuer l'étape 3 de pressage après l'étape 2 de broyage.

Cette étape 3 de pressage permet donc l'obtention d'un jus de presse et d'un gâteau de presse.

**• Etape 4 : hydrolyse enzymatique**

La pâte humide issue de l'étape 2 de broyage ou le gâteau de presse issu de l'étape 3 de pressage est placé dans une cuve d'hydrolyse avec de l'eau.

Optionnellement, et comme cela sera décrit ci-après, la fraction protéique issue de l'étape 12 de séparation, peut être réintroduite dans cette étape 4 d'hydrolyse enzymatique, en la mélangeant au gâteau de presse.

De manière optionnelle et dans le cas où l'eau de l'ébouillantage ne contient pas d'oxydant, l'eau de l'ébouillantage peut être récupérée et réintroduite lors de l'étape d'hydrolyse. En effet, cette eau contient des fractions d'insectes solubilisées de par l'action de cet ébouillantage et l'utilisation de celle-ci lors de l'hydrolyse permet de réduire les pertes. Optionnellement, cette eau issue de l'ébouillantage peut être dégraissée, certaines cires pouvant s'être dissoutes dans l'eau.

La quantité d'eau introduite à cette étape 4 d'hydrolyse enzymatique est similaire à celle introduite lors de l'étape 1 d'ébouillantage : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

L'hydrolyse enzymatique est réalisée avec une protéase, telle qu'une protéase commerciale pendant 4 à 8h, plus particulièrement pendant 4 à 5h, à un pH de 6 à 8, plus particulièrement de 6,5 à 7,5, à une température de 40 à 60°C, plus particulièrement de 45 à 55°C.

La quantité d'enzymes introduite lors de l'étape d'hydrolyse est inférieure à 10 % en poids sur le poids total de matière sèche estimé entrant en hydrolyse, préférentiellement inférieure à 6%, plus préférentiellement de l'ordre de 2 %.

L'hydrolyse protéolytique engendre la production d'une phase soluble contenant les peptides, glucosamines et oligochitines et d'un résidu solide formé de chitine, principalement de copolymère chitine-polypeptides.

### • Etape 5 : inactivation thermique

Afin de stopper l'activité des enzymes de la réaction et de stabiliser la phase soluble de l'hydrolyse, on réalise une inactivation thermique en chauffant ce jus entre 80 et 105° pendant 10 à 25 min, préférentiellement, 15 à 20 minutes. Selon un mode opératoire, cette étape 5 d'inactivation thermique est réalisée selon les techniques habituelles de stérilisation de l'industrie agroalimentaire. Selon un autre mode opératoire, l'inactivation enzymatique est réalisée par chauffage sous rayonnement IR ou UV, ou micro-ondes.

### • Etape 6 : pressage

Le résidu solide, majoritairement composé de chitine, est récupéré puis pressé par un pressoir afin d'essorer au maximum ce résidu pour réinjecter dans la phase soluble ce pressât. Le résidu pressé ainsi formé est composé essentiellement de chitine, principalement sous la forme de copolymère chitine-polypeptides.

### • Etapes (facultatives) 7 et 8 : lavage et séchage

Le résidu solide est ensuite lavé, filtré, à nouveau lavé puis séché par les technologies classiques connues par l'homme de métier.

Avantageusement, le système de séchage est étudié pour protéger la structure du copolymère de chitine-polypeptides : l'hydrométrie, la ventilation et la composition de l'air sont contrôlés. Avantageusement, le séchage peut s'effectuer dans une étuve ventilée à une température de 60 à 80°C, préférentiellement de l'ordre de 70°C.

Optionnellement, ces étapes peuvent comporter une étape de délipidation terminale : le résidu solide est traité avec du HCl afin d'enlever les derniers résidus lipidiques, notamment les cires cuticulaires.

Les étapes 9 à 11 qui suivent, visent à transformer la chitine en chitosan et ne sont donc mises en oeuvre que lorsque le produit souhaité est le chitosan.

### • Etape 9 (facultative) : broyage

Le résidu solide séché, comportant majoritairement de la chitine, est ensuite broyé, par exemple dans un broyeur à couteaux.

La production de chitosan à partir de chitine, par la réaction de désacétylation, dépend en grande partie de la taille des particules de chitine. Ainsi un broyage très fin du résidu solide séché avant désacétylation permet d'augmenter significativement les rendements et la vitesse de la réaction de désacétylation, comme cela est illustré dans le Tableau 1 ci-après :

**Tableau 1 : Efficacité de la désacétylation selon le broyage préalable de la chitine**

|  | Broyage 30 s | Broyage 45 s | Broyage 60 s | Broyage 120 s |
|---|---|---|---|---|
| 50% des particules | < 174 $\mu$m | < 117 $\mu$m | < 95 $\mu$m | < 67 $\mu$m |
| 90% des particules | < 310 $\mu$m | < 244 $\mu$m | < 157 $\mu$m | < 159 $\mu$m |
| DA* | 99% | 90% | 85% | 80% |
| *Mesure du Degré d'Acétylation DA | | | | |

Les conditions de la désacétylation effectuée dans l'essai rapporté dans le Tableau 1 sont les suivantes : 4h de réaction, 100°C, NaOH en solution aqueuse à 30% en volume, dans un ratio chitine estimée : solution de NaOH égal à 1:20.

Par conséquent, le résidu solide est préférentiellement broyé à une taille de particules inférieure à 200 $\mu$m,

plus préférentiellement, inférieure à 160 $\mu$m.

#### • Etape 10 : désacétylation

Le résidu solide, éventuellement broyé à l'étape 9, est ensuite placé dans un réacteur où est ajoutée une solution de soude concentrée pendant 4 à 24h, et préférentiellement 6 à 18h. L'hydroxyde de sodium en solution aqueuse à une teneur allant de 30% à 40% est ajouté selon un ratio poids en g de chitine broyée / volume en mL d'hydroxyde de sodium en solution aqueuse compris entre 1 :50 à 1 :10, de préférence de l'ordre de 1 :20. La cuve est ensuite chauffée, la température de désacétylation se situant entre 80 et 150°C, de préférence entre 90 et 120°C et plus préférentiellement à 100°C.

On obtient ainsi du chitosan en poudre.

Le chitosan peut ensuite subir toute opération connue de l'homme du métier permettant de le fonctionnaliser, notamment par l'ajout de radicaux (carboxylation, hydroxylation...)

#### • Etape 11 (facultative) : séchage

La poudre de chitosan est ensuite séchée entre 30 et 80°C, de préférence entre 50 et 70°C et de préférence à environ 60°C, afin d'obtenir une poudre ayant une teneur en matière sèche supérieure à 85%, plus particulièrement supérieure à 90%.

Les étapes qui suivent visent à récupérer une fraction huileuse et une fraction protéique à partir du jus obtenu à l'étape 3 de pressage et ne sont donc mises en oeuvre que lorsque qu'une telle récupération est souhaitée.

#### • Etape 12 : séparation

Le jus de presse subit une ou plusieurs étapes de séparation, afin de séparer la fraction huileuse (huiles des insectes) de la fraction protéique (protéines de l'hémolymphe des insectes). Ces étapes peuvent être réalisées par toute technologie de séparation des huiles bien connue de l'homme du métier, telle que la centrifugation, la décantation, la séparation par osmose inverse, l'ultrafiltration, le $CO_2$ supercritique, etc. ou une combinaison de plusieurs de ces technologies.

La séparation de la fraction huileuse peut être complexe, étant donné la présence d'huiles de compositions très différentes chez les insectes, les acides gras pouvant présenter de courtes chaines (C2-C5) ainsi que de très longues chaines (par exemple, pour les cires : > C25). La montée en température au-dessus du point de fusion de ces huiles (environ 38°C) lors de la centrifugation permet de solubiliser cette crème et de faciliter la séparation de la fraction huileuse du reste du jus. Le centrifugat passe ensuite en décantation selon un mode opératoire (type décanteur ou tricanteur), afin de séparer au mieux les huiles et les protéines.

Ces étapes permettent ainsi l'obtention d'une fraction huileuse.

La fraction protéique, une fois séparée de la fraction huileuse, peut être mélangée au gâteau de presse issu de l'étape 3 de pressage juste avant l'étape 4 d'hydrolyse. En effet, souvent plus de 20% des protéines sont perdues dans le jus lors de l'étape 3 de pressage, d'où l'intérêt de récupérer cette fraction et de la soumettre à l'étape d'hydrolyse.

#### • Etape 13 (facultative) : concentration

Selon un mode opératoire, la concentration est réalisée par évaporation sous vide de la partie aqueuse. Le concentrât présente un extrait sec supérieur à 10%, de préférence supérieur à 20%. Cette opération facilite le séchage et des additifs communément employés pour la conservation et la stabilité du produit peuvent être ajoutés à cette étape.

#### • Etape 14 (facultative) : séchage

Le concentrât est enfin séché par des technologies connues de l'homme du métier, telles que par exemple, par pulvérisation / atomisation (« spray-drying »), qui permet d'obtenir un extrait, c'est-à-dire une poudre sèche de concentrât riche en peptides et glucosamines, les glucosamines étant en particulier issues de l'hydrolyse partielle de la chitine par $H_2O_2$ (essentiellement).

D'autres caractéristiques et avantages de l'invention apparaîtront dans les exemples qui suivent, donnés à titre illustratif, avec référence aux figures, qui représentent respectivement :

- La Figure 1 est un schéma d'un mode de réalisation préférentiel du procédé selon l'invention,
- La Figure 2 un diagramme comparant le degré de pureté de la chitine obtenue par un procédé enzymatique comportant une ou plusieurs étapes préalables de broyage(s) et pressage,

- La Figure 3 est un diagramme comparant le taux de lipides mesuré dans différentes fractions du produit intermédiaire dont la chitine a été extraite,
- La Figure 4 illustre la répartition des lipides dans le jus et le gâteau de presse obtenus par un procédé enzymatique comportant des étapes préalables de broyage et de pressage ou une étape préalable de pressage,
- La Figure 5 est un diagramme illustrant la répartition des protéines/peptides selon leurs masses molaires (en %) dans un hydrolysat selon l'invention et dans le jus de presse,
- La Figure 6 est un diagramme illustrant la répartition relative des acides aminés constitutifs d'un l'hydrolysat selon l'invention,
- La Figure 7 est un diagramme illustrant l'abondance relative des acides aminés dans la chitine ;
- La Figure 8 : Effet du pressage sur le taux de cendres dans l'hydrolysat - différents insectes,
- La Figure 9 : Effet du pressage sur le taux de cendres dans l'hydrolysat - différentes enzymes,
- La Figure 10 : Taux de cendres dans l'hydrolysat avec le procédé broyage + pressage - différents insectes,
- La Figure 11 : Abondance relative de protéines de grande taille en fonction de différents procédés et différents insectes pour une hydrolyse effectués avec la Prolyve,
- La Figure 12 : Abondance relative de protéines de grande taille en fonction de différents procédés et différentes enzymes appliquées à *T. molitor,*
- La Figure 13 : Effet du pressage sur la teneur en protéines de l'hydrolysat - différentes enzymes,
- La Figure 14 : Effet du pressage sur la teneur en protéines de l'hydrolysat - différents insectes,
- La Figure 15 : Effet du pressage sur la teneur en lipides de l'hydrolysat - différentes enzymes,
- La Figure 16 : Effet du pressage sur la teneur en lipides de l'hydrolysat - différents insectes,
- La Figure 17 : Digestibilité pepsique des hydrolysats obtenus - provisoire,
- La Figure 18: TM + prolyve : abondance relative des acides aminés de l'hydrolysat,
- La Figure 19 : TM + sumizyme : abondance relative des acides aminés de l'hydrolysat,
- La Figure 20 : TM + novozyme : abondance relative des acides aminés de l'hydrolysat,
- La Figure 21 : TM + neutrase : abondance relative des acides aminés de l'hydrolysat,
- La Figure 22 : HI + prolyve : abondance relative des acides aminés de l'hydrolysat,
- La Figure 23 : GM + prolyve : abondance relative des acides aminés de l'hydrolysat,
- La Figure 24 : AD + prolyve : abondance relative des acides aminés de l'hydrolysat,
- La Figure 25 : Effet du pressage sur le taux de cendres dans la chitine - différentes enzymes,
- La Figure 26 : Taux de cendres dans la chitine de différents insectes,
- La Figure 27 : Teneur en lipides dans la chitine,
- La Figure 28 : Taux d'acides aminés dans la chitine,
- La Figure 29 : TM + prolyve : abondance relative des acides aminés de la chitine,
- La Figure 30 : TM + sumizyme : abondance relative des acides aminés de la chitine,
- La Figure 31 : TM + novozyme : abondance relative des acides aminés de la chitine,
- La Figure 32 : TM + neutrase : abondance relative des acides aminés de la chitine,
- La Figure 33 : HI + prolyve : abondance relative des acides aminés de l'hydrolysat,
- La Figure 34 : GM + prolyve : abondance relative des acides aminés de l'hydrolysat,
- La Figure 35 : AD + prolyve : abondance relative des acides aminés de l'hydrolysat,
- La Figure 36 : Pureté gravimétrique de la chitine en fonction de différents procédés et différentes enzymes appliquées à *T. molitor,*
- La figure 37 : Pureté gravimétrique de la chitine en fonction de différents procédés et différents insectes avec la Prolyve,
- La Figure 38 : Pureté colorimétrique de la chitine en fonction de différents procédés et différentes enzymes avec *T. molitor,*
- La Figure 39 : Pureté colorimétrique de la chitine en fonction de différents procédés et différents insectes avec la Prolyve,
- La Figure 40 : Pureté par différence de la chitine,
- La Figure 41 : Degré d'acétylation de la chitine obtenue par le procédé pour T. *molitor,*
- La Figure 42 : Degré d'acétylation de la chitine obtenue par le procédé avec la Prolyve,
- La Figure 43 : Représentation de la cuticule par microscopie par fluorescence bi-photon,
- La Figure 44 : Degré de cristallinité des chitines obtenues, et
- La Figure 45 : Taille des protéines dans l'hydrolysat.

**Exemple 1 : Exemple de procédé selon l'invention**

600 g de larves de *T. molitor* sont introduits dans un bêcher, placé dans un bain-marie à 100 °C et contenant 600 mL d'eau, préalablement portée à ébullition. Après 5 minutes, le bécher est retiré du bain-marie, les larves sont essorées, puis mixées avec un volume d'eau de 600 mL. Le liquide ainsi obtenu est pressé avec une presse bi-vis de type Angel dans les conditions suivantes :

- Vitesse = 82 tr/min;
- W (énergie) = 3 HP (chevaux vapeur) soit 2,68 x $10^6$ J;
- Porosité (approximative) = 0,5 mm dans la première partie et 0,2 mm dans la dernière partie.

On obtient ainsi un jus de presse et un gâteau de presse de 136,49 $\pm$ 4,48 g en poids humide, dont 100,22 $\pm$ 0,22 g sont utilisés dans les étapes suivantes. Tout autre type de presse aurait toutefois pu être utilisé, dans la mesure où celle-ci permet l'extraction l'obtention d'un gâteau de presse sensiblement similaire en termes de teneur en eau et/ou de teneur en lipides. A titre d'exemple, d'autres essais ont été réalisés avec le filtre presse de type Choquenet présentant les caractéristiques suivantes :

- Surface de filtration de la cellule = 50 $cm^2$ ;
- Pression = 2 à 8 bars ;
- Température = 20 à 80 °C,
- Porosité = 25 à 80 $\mu$m ;
- Débit en fin de filtration = 100 à 250 mL/h.

Le gâteau de presse est transféré dans un Erlen Meyer contenant 600 mL d'une solution de protéase (Prolyve NP conc) à 1% (par rapport au poids humide du gâteau de presse), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 16,99 $\pm$ 1,77 g de chitine par ce procédé. L'hydrolysat (filtrat obtenu après hydrolyse) représente dans ces conditions 609,98 $\pm$ 10,9 g avec une teneur en matière sèche de 5,05%, ce qui, après lyophilisation, donne 30,8 $\pm$ 0,55 g de l'hydrolysat sec.

**Exemple 2 : Influence des étapes mécaniques préalables à l'hydrolyse enzymatique sur le degré de pureté de la chitine obtenue**

Différents types de prétraitement mécanique ont été testés, le broyage (« broyage 1 ») seul, le broyage suivi du pressage, le broyage suivi du pressage et d'un second broyage (« broyage 2 »), ainsi que le pressage seul.
Pour le pressage, la presse de type Angel décrite à l'exemple 1 a été utilisée.

1. Matériel et méthodes

Production de chitine avec un broyage

200 g de larves de *T. molitor* sont introduits dans un bêcher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est transféré dans un Erlen Meyer contenant 2 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 8,13 $\pm$ 0,27 g de chitine par ce procédé.

Production de chitine avec un broyage suivi d'un pressage

200 g de larves de *T. molitor* sont introduits dans un bêcher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est passé dans une presse de type bi-vis. 30 g du gâteau de presse ainsi obtenu sont transférés dans un Erlen Meyer contenant 150 mL d'eau et 0,3 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 4,71 $\pm$ 0,11 g de chitine par ce procédé.

Production de chitine avec un premier broyage (« broyage 1 ») suivi d'un pressage et d'un second broyage (« broyage 2 »)

200 g de larves de *T. molitor* sont introduits dans un bêcher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est passé dans une presse de type bi-vis. Le gâteau de presse ainsi obtenu est séché 24 heures dans une étuve à 70 °C, puis broyé à 250 $\mu$m. 10 g de la poudre ainsi obtenue sont transférés dans un Erlen Meyer contenant 50 mL d'eau et 0,1 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 4,93 $\pm$ 0,12 g de chitine par ce procédé.

Production de chitine avec pressage uniquement

200 g de larves de *T. molitor* sont introduits dans un bêcher, placé dans un bain-marie à 100 °C et contenant 200 mL d'eau préalablement portée à ébullition. Après 5 minutes, le bécher est retiré du bain-marie, les larves sont essorées, puis passées dans une presse de type bi-vis. 90 g de gâteau de presse ainsi obtenu sont transférés dans un Erlen Meyer contenant 450 mL d'eau et 0,9 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 6,48 $\pm$ 0,28 g de chitine par ce procédé.

Production de chitine par voie chimique

50 g de larves de *T. molitor* sont introduits dans un bêcher, placé dans un bain-marie à 100 °C et contenant 50 mL d'eau, préalablement portée à ébullition. Après 5 minutes, le bécher est retiré du bain-marie, les larves sont essorées, puis mixées avec un volume d'eau de 60 mL. Le liquide ainsi obtenu est transféré dans un récipient de 1 L et 500 mL d'une solution d'HCl 1M sont ajoutés. L'ensemble est placé à 90 °C sous agitation pendant 1 heure. Le contenu est ensuite filtré et le résidu solide est transféré dans un flacon de 1 L contenant 500 mL d'une solution de NaOH 1M, l'ensemble est placé à 90 °C sous agitation pendant 24 heures. Le résidu est ensuite filtré et placé en étuve ventilée à 70 °C pendant 24 heures. On obtient ainsi 0,944 $\pm$ 0,005 g de chitine purifiée par voie chimique.

Calcul du degré de pureté

Le degré de pureté de la chitine est déterminé en comparaison de la masse en résidu sec obtenue par rapport à celle résultant d'une extraction chimique, 5 % environ de la matière sèche initiale.

Mesure du taux de lipides

On place 2 g d'échantillon dans un bêcher, on y ajoute 0,2 g de $Na_2SO_4$ et 15 mL de $CHCl_3$/MeOH (2/1 v/v). L'ensemble est placé sous agitation magnétique pendant 20 minutes, ensuite la solution est filtrée, le résidu est placé de nouveau dans le bécher avec 10 mL de $CHCl_3$/MeOH (2/1 v/v). L'ensemble est placé sous agitation magnétique pendant 15 minutes, ensuite la solution est filtrée, les phases solvants sont réunies et évaporées à poids constant. La teneur en lipides est déterminée comme pourcentage de masse après extraction-évaporation par rapport à la masse initiale de l'échantillon (2 g).

2. Résultats

Comme cela peut être constaté sur la Figure 2, le procédé a une influence sur la pureté de la chitine obtenue, les meilleurs résultats étant obtenus avec un pressage a minima. Le meilleur résultat est obtenu avec le broyage suivi du pressage, à savoir une chitine présentant un degré de pureté de 78% et le moins bon avec le broyage seul, à savoir une chitine présentant un degré de pureté de 48%.

Une analyse plus fine du produit intermédiaire dont la chitine a été extraite, permet de constater qu'un bas taux de lipides est favorable à une plus grande pureté de la chitine obtenue (Figure 3). Par « produit intermédiaire », on vise le produit entrant en hydrolyse enzymatique, c'est-à-dire issu de la dernière étape avant hydrolyse, à savoir, selon les procédés de production ci-avant, l'étape de broyage 1 ou 2 ou l'étape de pressage.

L'analyse du taux des lipides dans la chitine et le jus d'hydrolyse permet en effet de relever qu'en fonction du taux de lipides initiaux présents dans le produit intermédiaire, le taux des lipides dans la chitine est relativement stable, de 7 à 15%, tandis que le taux des lipides dans l'hydrolysat varie, quant à lui, de 11 à 47% (Figure 3).

Plus particulièrement, si le produit intermédiaire a une teneur en lipides de 35%, alors :

- la chitine qui résultera de l'hydrolyse ne sera pure qu'à 50% et comportera 10% lipides, et
- la teneur en lipides de l'hydrolysat avoisinera quant à elle les 40%.

En revanche, si la teneur en lipides du produit intermédiaire est de 7%, alors :

- la chitine obtenue après hydrolyse aura une pureté de 80% et aura une teneur en lipides de 8% et l'hydrolysat présentera également une faible teneur en lipides, de l'ordre de 10%.

Cela indique que lorsque le taux des lipides initiaux est élevé, supérieur à 12%, l'enzyme responsable d'hydrolyse est amenée à hydrolyser non seulement les protéines, mais également les lipides par promiscuité catalytique. C'est ainsi qu'un taux de lipides dans la chitine similaire est obtenu, à savoir 8,6 et 7,9% dans les cas où les lipides initiaux étaient de 35 et 7% respectivement. En revanche, la pureté de la chitine passe dans ce cas de 48 à 84% respectivement. Ainsi, sur les 52% d'impuretés d'une part et 16% de l'autre, 8% en moyenne sont dus aux lipides, la quantité de protéines restées accrochées à la chitine est donc de 38 points supérieure dans le cas où davantage de lipides étaient présents dans le produit intermédiaire soumis à l'hydrolyse.

Finalement, l'importance du broyage en amont du pressage peut également être étudiée (Figure 4). Il apparait ainsi clairement que la répartition des lipides entre le gâteau et le jus de presse est bien plus efficace, 12,9 *versus* 87,1 contre 42,7 *versus* 57,3, lorsqu'un broyage préalable a été réalisé.

### Exemple 3 : **Analyse de l'hydrolysat**

Une analyse détaillée de l'hydrolysat obtenu à l'exemple 1 a été effectuée.

### 1. Teneur en glucosamines

La teneur de l'hydrolysat en glucosamines et certains autres sucres a été analysée par chromatographie gazeuse après méthanolyse et sillilation.

Mode opératoire :

10 mg de l'échantillon et 50 μg d'étalon interne sont placés dans 500 μL d'un mélange méthanol/acide chlorhydrique 3 N pendant 4 heures à 110 °C (ou 24 heures à 130 °C). Le mélange est ensuite neutralisé avec le $Ag_2CO_3$. 50 μL d'anhydride acétique sont ajoutés afin de ré-acétyler les osamines éventuellement présentes. Après une nuit à l'obscurité et à température ambiante, les échantillons sont centrifugés (15 min, 3000 rpm) et le surnageant est évaporé. Les composés sont ensuite dissous dans 100 μL de pyridine et incubés une nuit à température ambiante avec 100 μL de BSTFA (Supelco). Les réactifs sont ensuite évaporés et le résidu repris dans 700 μL de $CH_2Cl_2$ et injecté en CPG.

Programme de température: 1 minute à 120 °C, rampe de 1.5 °C/minute jusqu'à 180 °C, puis 2 °C/minute jusqu'à 200 °C.

Détection : FID

Colonne : HP-5MS (30 m, 0.25 mm diamètre interne)

Etalon interne : myo-inositol

Les différentes teneurs ont été mesurées et calculées de deux façons différentes (Tableau 2). Il en ressort que le glucosamine est contenu dans l'hydrolysat à hauteur de 0,1-0,15 % en masse et avec un ratio de 0,04-0,05 par rapport au glucose.

**Tableau 2 : Répartition du glucosamine, mannose et glucose dans l'hydrolysat**

|  | Méthanolyse à 110 °C | | Méthanolyse à 130 °C | |
|---|---|---|---|---|
|  | % massique | Ratio molaire | % massique | Ratio molaire |
| Glucose | 1,6 ± 0,3 | 1 | 2 ± 0,4 | 1 |
| Mannose | 0,3 ± 0,05 | 0,15 ± 0,005 | 0,4 ± 0,08 | 0,15 ± 0,007 |
| Glucosamine | 0,1 ± 0,02 | 0,04 ± 0,007 | 0,15 ± 0,04 | 0,05 ± 0,003 |

### 2. Taille des protéines

La taille des protéines/peptides de l'hydrolysat a été évaluée par HPLC, appareil Shimadzu 20A, à température ambiante, sur colonne Superdex Peptide 10/300 GL, dans le tampon acétonitrile (ACN) 30%, acide trifluoroacétique (TFA) 0,1%, avec un débit de 0,4 mL/min. La détection a été réalisée à 205 nm et le volume d'échantillon injecté était de 20 μL.

De même, la taille des protéines/ peptides du jus de presse préparé à l'Exemple 1, a également été évaluée dans des conditions identiques à des fins de comparaison.

La comparaison de la taille des protéines entre le jus de presse et l'hydrolysat (Figure 5) montre clairement que l'essentiel, 76%, des peptides présents dans l'hydrolysat ont une masse molaire comprise entre 130 et 900 Da. En revanche, la partie des protéines peu digestes (de masse molaire supérieure à 1300 Da) est réduite de 31% à 2%. De même, la proportion de peptides présentant un caractère d'amertume (de masse molaire inférieure à 130 Da) passe de 38% à 15%. Les critères de digestibilité et palatabilité se trouvent donc ainsi améliorés par le traitement enzymatique proposé.

### 3. Digestibilité

Le taux de digestibilité pepsique de l'hydrolysat est estimé à 99,6 % des protéines totales. Il a été mesuré par un laboratoire externe, la méthode utilisée est conforme à la directive 72/199/EEC et a été pratiquée sans dégraissage.

### 4. Teneur en protéines

Le taux de protéines de l'hydrolysat est estimé à 84,8 %. Il a été mesuré par un laboratoire externe, selon la méthode Kjeldahl avec le coefficient de correction de 6,25. La méthode utilisée est conforme au règlement CE 152/2009.

### 5. Teneur en lipides

Le taux de lipides de l'hydrolysat est estimé à 0,7 ± 0,5 %. Il a été mesuré par un laboratoire externe selon la méthode dite de « l'hydrolyse » adaptée du règlement CE 152/2009 (procédé B).

### 6. Composition en acides aminés

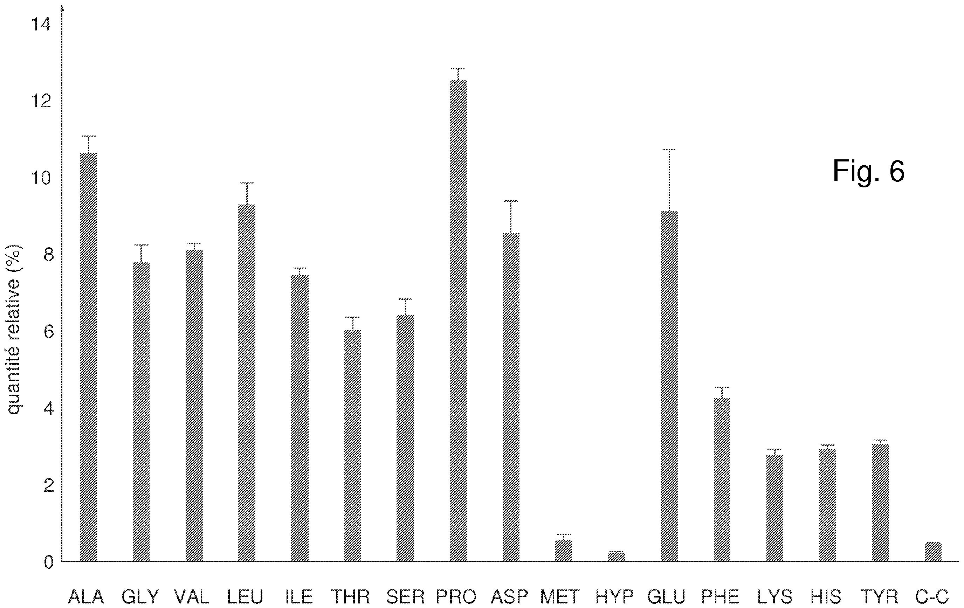

L'hydrolysat obtenu après traitement par les différentes enzymes a été analysé quant à sa composition en acides aminés (Figure 6). On y observe la prépondérance de la proline, conjointement avec la présence de l'hydroxy proline (HYP) - métabolite de la proline et acide aminé absent chez certains organismes, par exemple les crustacés. Or, la proline et son métabolite, l'hydroxy proline, jouent un rôle essentiel dans le métabolisme, notamment en permettant la synthèse d'autres acides aminés tels que l'arginine et le glutamate. Si la plupart des mammifères sont capables de synthétiser la proline, la production de cet acide aminé par les nouveau-nés, les oiseaux et les poissons est insuffisante, c'est pourquoi une supplémentation en proline et hydroxy proline est parfois mise en place afin d'accroitre la croissance de certains animaux. Par ailleurs, la proline est le premier acide aminé contenu dans le lait des mammifères, elle y est présente à hauteur de 12%, en revanche son contenu dans les protéines des plantes est bien moindre, seulement 2,9% dans le soja et 0,8% dans le maïs. On retrouve donc dans cet hydrolysat une teneur en proline aussi élevée que dans le lait et bien supérieure à celle trouvée dans les protéines végétales.

Les autres acides aminés présents en quantité importante sont l'alanine, la leucine et le glutamate (avec la glutamine). Leurs quantités relatives sont supérieures à 9%. En revanche, les acides aminés soufrés tels que la cystéine et la méthionine sont en faible quantité, de l'ordre de 0,5%.

La méthode utilisée pour la détermination de ces résultats (hydrolyse acide) n'a pas permis la mise en évidence des acides aminés tels que le tryptophane et l'arginine. Par ailleurs, l'asparagine a été entièrement transformée en acide aspartique et la glutamine en acide glutamique et ce qui est observé sous les pics ASP et GLU est en fait respectivement la somme de l'acide aspartique et de l'asparagine d'une part et de l'acide glutamique et de la glutamine d'autre part, initialement présents dans l'hydrolysat.

### Exemple 4 : Analyse de la chitine

Une analyse détaillée en acides aminés de la chitine obtenue à l'exemple 1 a été effectuée.

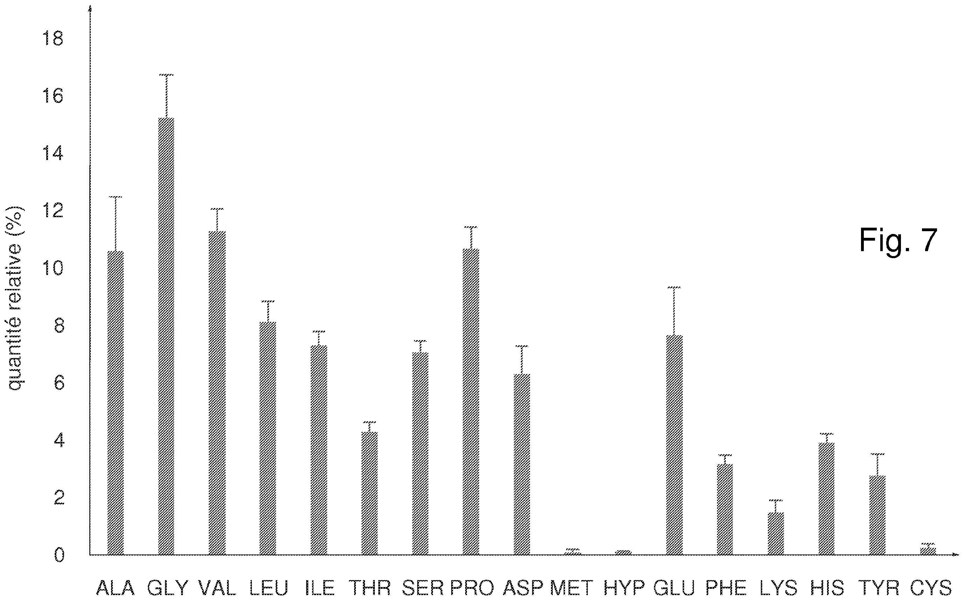

La teneur totale en acides aminés est de 32,3 g pour 100 g de copolymère (déterminée comme somme des acides aminés). Les acides aminés majoritairement présents sont la valine, la glycine, l'alanine et la tyrosine (Figure 7).

Les analyses ont été sous-traitées et les résultats obtenus selon la méthode NF EN ISO 13904 pour le tryptophane et NF EN ISO 13903 pour les autres acides aminés.

**Exemple 5** : **Caractérisation de l'hydrolysat et de la chitine selon le procédé de production mis en œuvre**

**I. Matériel et méthodes**

a) Matériel

Insectes

Différents insectes ont été étudiés :

- un coléoptère : *Tenebrio molitor* (*T. molitor* ou TM),
- un lépidoptère : *Galleria mellonella* (*G. melonella* ou GM),
- un diptère : *Hermetia illucens* (*H. illucens* ou HI), et
- un orthoptère : *Acheta domesticus* (*A. domesticus* ou AD).

Enzymes

Différentes enzymes ont été mises en oeuvre lors de l'étape d'hydrolyse.
Cette mesure de l'activité enzymatique est basée sur le principe de la mesure de libération de la tyrosine à 275 nm lors de l'hydrolyse de la caséine par une enzyme protéolytique (Valley research SAPU Assay method, par Karen PRATT).

$$\frac{SAPU}{g} = \frac{(\Delta A - i) \times 11}{m \times 30 \times C \times 1}$$

SAPU/g = une unité spectrophotométrique de protéase
$\Delta A$ = absorbance corrélée
i = ordonnée à l'origine
11 = volume final de réaction
M = coefficient directeur de la courbe de calibration
30 = temps de réaction (en minutes)
C = concentration de l'enzyme (g/mL) dans la solution enzymatique ajoutée
1 = 1 mL volume de la solution d'enzyme ajoutée

La courbe de calibration est obtenue par mesure de l'absorbance des solutions de tyrosine de différentes concentrations dans un tampon phosphate (0,2 M, pH 7).
5 mL d'une solution de caséine (0,7% m/v, tampon phosphate 0,2 M, pH 7, chauffée pendant 30 minutes à 90 °C et additionnée de 3,75 g/L$_{solution}$) sont incubés avec 1 mL de la solution enzymatique (0,15 g dans 100 mL du tampon glycine, 0,05M) à tester à 37 °C pendant 30 minutes. On y ajoute ensuite 5 mL d'une solution de TCA (18 g TCA, 11,35 g d'acétate de sodium anhydre, 21 mL d'acide acétique glacial, complétée avec l'eau déminéralisée à 1 litre de solution), mélange sur un vortex, filtre et mesure l'absorbance à 275 nm.
Le blanc est préparé de façon identique mais sans ajout d'enzyme, 1 mL d'eau déminéralisée est ajouté à la place afin de retrouver le même volume réactionnel.
Les activités ainsi mesurées pour les différentes enzymes utilisées (Prolyve NP, Novozyme 37071, Neutrase et Sumizyne) sont répertoriées dans le Tableau 3.

| enzyme | Prolyve | Novozyme | Neutrase | Sumizyme |
|---|---|---|---|---|
| **Activité enzymatique voulue** | 3789,52 | 3789,52 | 3789,52 | 3789,52 |
| **Activité enzymatique / g** | 3789,52 | 1662,35 | 2213,24 | 3237,19 |
| **m (g)** | 1,00 | 2,28 | 1,71 | 1,17 |

**Tableau 3.** Correspondance des activités et masses enzymatiques des enzymes utilisées

b) Procédés de production

Procédé de production avec broyage seul (désigné « broyage » dans les figures)

600 g d'insectes (qu'il s'agisse de larves dans le cas de *T. molitor, G. melonella* ou *H. illucens,* ou de criquets dans le cas de *A. domesticus)* frais sont introduite dans une enceinte où ils sont abattus à la vapeur d'eau (115 °C, 5 minutes). Les insectes sont ensuite introduits dans un appareil de mixage et 75 mL d'eau pour 100 g d'insectes sont ajoutés, l'ensemble est ensuite mixé. 100 g (poids humide) de produit ainsi obtenu sont ensuite introduits dans un ballon tricol muni d'un réfrigérant et d'un agitateur mécanique, une enzyme protéolytique d'une activité équivalente à 3789 SAPU est alors ajouté. La réaction est ensuite portée à 45 °C pendant 4 heures. La température est ensuite portée à 90 °C pendant 15 minutes, le mélange réactionnel est enfin filtré (0,40 - 0,45 μm). Le résidu est séché pendant 24 heures à 70 °C : il s'agit donc de la chitine obtenue par voie enzymatique de purification ; le filtrat est congelé et est lyophilisé : il s'agit donc de l'hydrolysat.
Le procédé est identique quel que soit l'insecte ou l'enzyme étudié.

Procédé de production avec broyage suivi d'un pressage (désigné « broyage +pressage » dans les figures)

600 g d'insectes (qu'il s'agisse de larves dans le cas de *T. molitor, G. melonella* ou *H. illucens,* ou de criquets dans le cas de *A. domesticus*) frais sont introduite dans une enceinte où ils sont abattus à la vapeur d'eau (115 °C, 5 minutes). Les insectes sont ensuite introduits dans un appareil de mixage et 75 mL d'eau pour 100 g d'insectes sont ajoutés, l'ensemble est ensuite mixé et pressé (presse bi-vis, ou filtre presse, ou autre système de pressage). 100 g (poids humide) de produit ainsi obtenu sont ensuite introduits dans un ballon tricol muni d'un réfrigérant et d'un agitateur magnétique, 500 mL d'eau sont ajoutés ainsi qu'une enzyme protéolytique d'une activité équivalente à 3789 SAPU. La réaction est ensuite portée à 45 °C pendant 4 heures. La température est ensuite portée à 90 °C pendant 15 minutes, le mélange réactionnel est enfin filtré (0,40 - 0,45 μm). Le résidu est séché pendant 24 heures à 70 °C : il s'agit donc de la chitine obtenue par voie enzymatique de purification ; le filtrat est congelé et est lyophilisé : il s'agit donc de l'hydrolysat.
Le procédé est identique quel que soit l'insecte ou l'enzyme étudiés.

c) Analyses

Mesure du taux de cendres

Le taux de cendres a été déterminé selon la méthode relevant du règlement CE 152/2009 du 27-01-2009.

Mesure de la taille des protéines

100 mg d'échantillon sont placés dans 10 mL de tampon phosphate /NaCl (pH 7,4, 0,137 mM). L'échantillon a été agité durant une minute (vortex), centrifugé à 900 g durant 1 min et filtré ensuite sur une membrane de 0,45 $\mu$m. L'analyse a été réalisée sur un système de chromatographie par exclusion stérique, avec la colonne Nucleogel GFC-300, l'éluent utilisé est le tampon phosphate /NaCl (pH 7,4, 0,137 mM), le débit est de 1,0 mL/min, la détection est réalisée par un détecteur UV à 280 nm.

### Mesure de la teneur en protéines

Le taux de protéines est obtenu par la méthode Dumas, avec le coefficient de conversion de 6,25, adaptée de la norme NF EN ISO 16634-1

### Mesure de la teneur en lipides

Le taux de lipides est obtenu par une méthode adaptée du règlement CE 152/2009 - procédé B - SN.

### Digestibilité pepsique

La digestibilité pepsique est mesurée par la méthode décrite dans la directive 72/199/CE.

### Abondance relative en acides aminés

L'abondance des acides aminés a été déterminée par une méthode issue du règlement CE 152/2009 du 27-01-2009 - SN. La teneur en tryptophane a été déterminée séparément par une méthode adaptée du règlement CE 152/2009 du 27-01-2009 - SN. L'abondance relative a été calculée en rapportant la teneur de chaque acide aminé au taux des acides aminés.

### Taux d'acides aminés

Le taux des acides aminés a été déterminé en additionnant les valeurs individuelles obtenues pour chaque acide aminé, y compris le tryptophane.

### Pureté gravimétrique

La pureté gravimétrique est déterminée en comparaison de la masse en résidu sec obtenue par rapport à celle résultant d'une extraction chimique, cette dernière étant évaluée à 5% environ de la matière sèche initiale.

### Pureté colorimétrique

La couleur de l'échantillon a été estimée grâce à l'analyse de photographies d'échantillons à l'aide du logiciel ImageJ selon les trois couleurs rouge, verte et bleue (RVB ou RGB en anglais), leur moyenne représentant une cotation de la couleur réelle. Un échantillon de chitine de crevettes commercialisé par Chitine France a été pris comme standard (100% de pureté) et la pureté colorimétrique des échantillons produits a été calculée en tant que pourcentage de cette couleur (ratio couleur de l'échantillon/couleur du standard).

### Pureté par différence

Dans le cas de cette mesure, les quantités d'impuretés connues (acides aminés, lipides et cendres) ont été soustraites à la valeur de pureté absolue (100%) pour obtenir la valeur de la pureté estimée par différence ; *i.e.* un échantillon qui contient 30% protéines, 10% de lipides et 1% de cendres, se voit dès lors attribuer une pureté de 100-30-10-1=59%

### Degré de cristallinité

Les mesures ont été effectuées selon la technique WAXS (wide angle X-ray scattering) sur l'appareil Bruker D8 Advance (A25 DaVinci design) équipé d'un détecteur Lynxeye XE. Les résultats ont été interprétés suivant le procédé décrit dans Loelovich, M. Res. Rev.: J. Chem. 2014, 3, 7-14.

Degré d'acétylation

Les mesures ont été réalisées à l'aide d'un appareil RMN $^{13}$C CP/MAS, de marque Bruker et équipé d'un aimant de 800 MHz.

L'analyse des résultats a été effectué conformément à celle décrite dans Simionatto Guinesi, L.; Gomes Cavalheiro, E. T. Thermochimica Acta 2006, 444, 128-133 et Heux, L.; Brugnerotto, J.; Desbrières, J.; Versali, M.-F.; Rinaudo, M. Biomacromolecules 2000, 1, 746-751.

Mesure de l'abondance atomique et fonctionnelle à la surface

Les mesures ont été réalisées à l'aide d'un appareil XPS Escolab 250, de marque VG Scientific et équipé d'une source RX Ka A1 (1486,6 eV), d'un monochromateur et d'une lentille magnétique.

Les échantillons, préalablement réduits en poudre, ont été placés sous vide pendant 48 à 72 heures et analysés ensuite.

Représentation de la cuticule par microscopie par fluorescence bi-photon

Microscopie multiphotonique sur les appareils DISCO (Synchrotron Soleil).

Lambda exc=810nm, power 18%, filtre bleu turquoise et ocre 406/15 (SHG), filtre bleu 460/60, filtre vert 550/88.

## II. Hydrolysat

a) Cendres

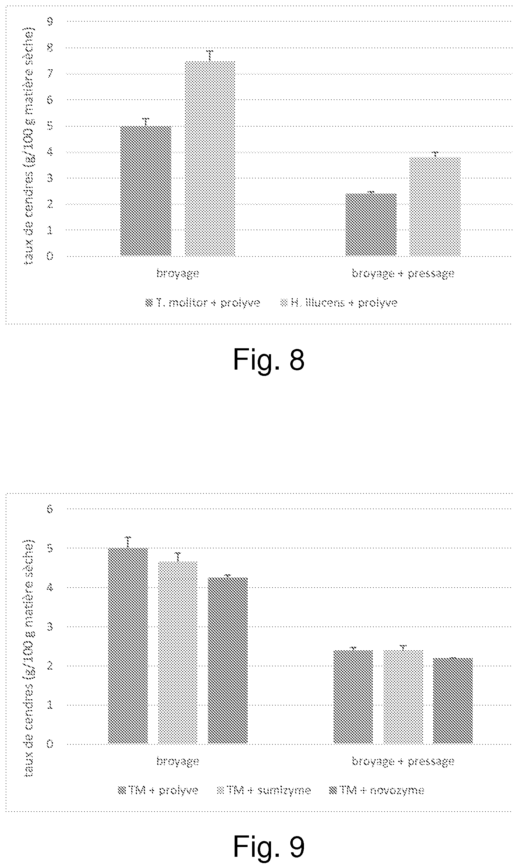

L'effet du pressage est très significatif sur le taux de cendres dans l'hydrolysat obtenu, et ce quel que soit l'insecte étudié (Figure 8). En effet, la diminution du taux de cendres peut atteindre 52%, la proportion à la baisse est relativement similaire lorsqu'il s'agit d'insectes naturellement riches ou pas en minéraux. Ainsi, dans le cas de *H. illucens* - le taux de cendres passe de 7,5 g/100 g de matière sèche lorsque le procédé avec broyage est utilisé à 3,8 g/100 g de matière sèche, lorsque l'on adjoint une étape de pressage, *i.e.* 49% de diminution ; dans le cas de *T. molitor,* on passe ainsi de 5 g/100 g à 2.4 g/100 g, i.e. 52% de diminution.

L'effet du pressage est également significatif sur le taux de cendres dans l'hydrolysat obtenu, et ce quel que soit l'enzyme mise en oeuvre (Figure 9). En particulier, on note que *T. molitor,* quel que soit l'enzyme, le taux de cendres dans l'hydrolysat est inférieur à 3% lorsque l'on utilise le procédé avec pressage.

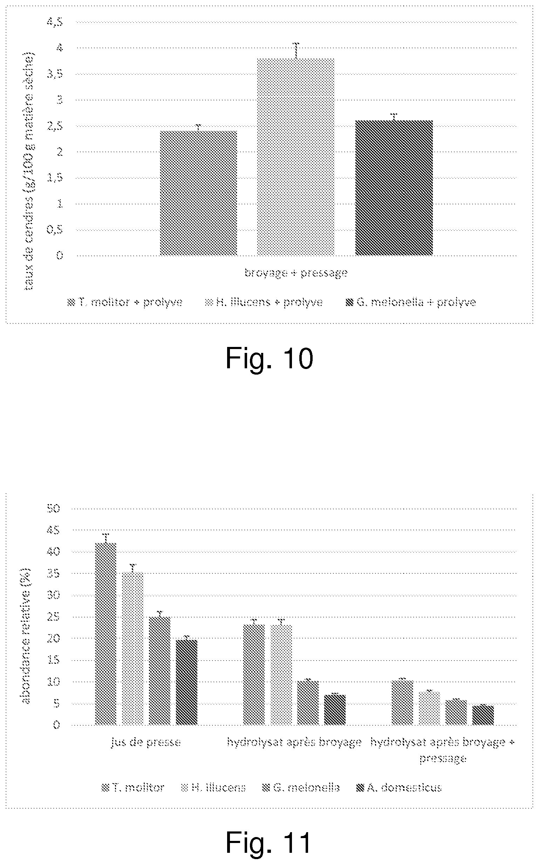

Le taux de cendres dans l'hydrolysat (obtenu par le procédé broyage + pressage) est inférieur à 4 g/100 g de matière sèche, et ce quelque soit l'insecte étudié (Figure 10).

b) Taille des protéines dans l'hydrolysat

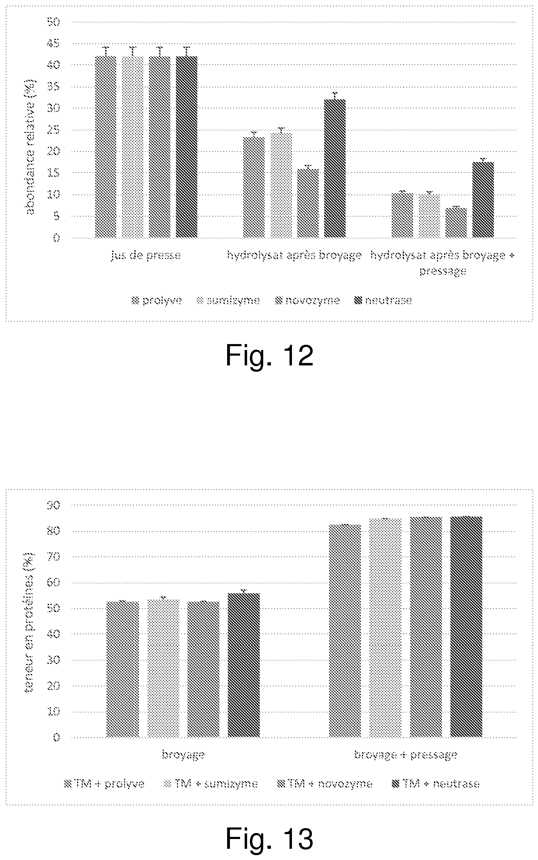

L'utilisation de l'étape de pressage permet clairement d'améliorer la performance des enzymes protéolytiques, et ceux quel que soit l'insecte (Figure 11) ou l'enzyme utilisés (Figure 12). L'abondance relative des protéines de grande taille chute ainsi significativement par rapport au procédé ne comportant que l'étape de broyage, l'hydrolysat final n'en comportant plus que 10,3% maximum dans le cas de l'hydrolyse effectuée avec la prolyve, et ce quel que soit l'insecte ; et pas plus que 17,5% dans le cas de l'hydrolyse de *T. molitor,* et ce quel que soit l'enzyme utilisée.

c) *Teneur en protéines*

La teneur en protéines de l'hydrolysat dépend très fortement du procédé utilisé. Ainsi lorsque le broyage est appliqué seul, les protéines ne représentent que 53,59 $\pm$ 1,5% de la matière sèche de l'hydrolysat de *T. molitor* ainsi obtenu, et ce quel que soit l'enzyme utilisée (Figure 13). En revanche, lorsque le broyage est suivi du pressage, le taux de protéines dans l'hydrolysat passe à 84,58 $\pm$ 1,4%, il subit ainsi une augmentation de 53 - 62%, suivant l'enzyme utilisée.

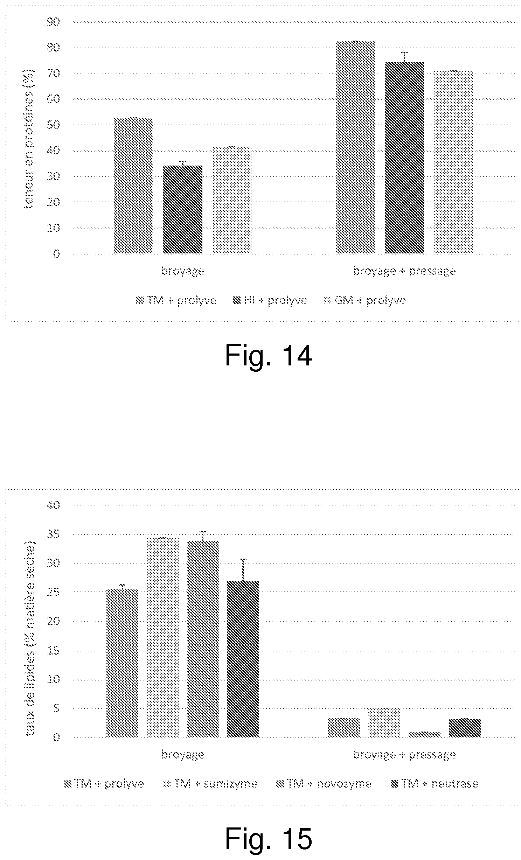

Cette augmentation est d'autant plus forte que l'insecte initialement était pauvre en protéines, ainsi dans le cas de *G. melonella,* le taux de protéines passe de 41,25% à 71%, subissant une augmentation de près de 86% et dans le cas de *H. illucens,* l'augmentation est même de 118%, puisque l'on passe de 34,2% à 74,5% (Figure 14).

d) *Teneur en lipides*

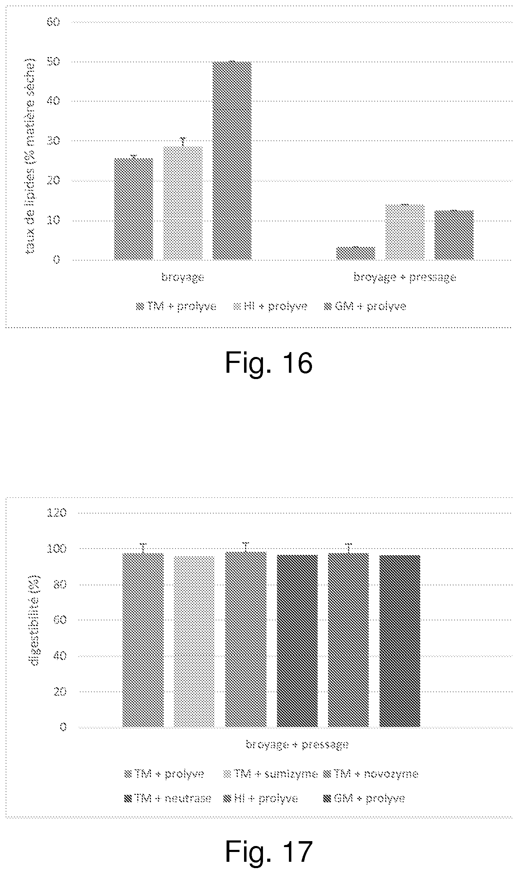

Le pressage a une influence majeure sur la teneur de l'hydrolysat en lipides et ce quel que soit l'enzyme (Figure 15) ou l'insecte étudié (Figure 16). En effet, la teneur en lipides diminue drastiquement de 51,4-97,7% passant ainsi de 28,6 à 13,9% dans le cas de *H. illucens* (51,4% de diminution) et de 33,85 à 0,9% dans le cas de *T. molitor* avec novozyme (97,3% de diminution).

e) Digestibilité pepsique

La digestibilité pepsique des hydrolysats ainsi obtenus est très élevée, supérieure à 96% quel que soit l'enzyme ou l'insecte étudié (Figure 17).

f) Abondance en acides aminés

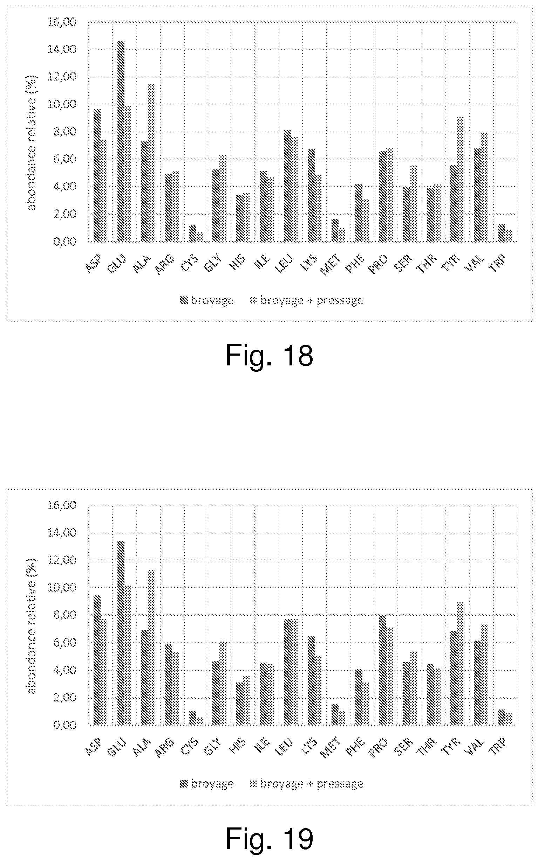

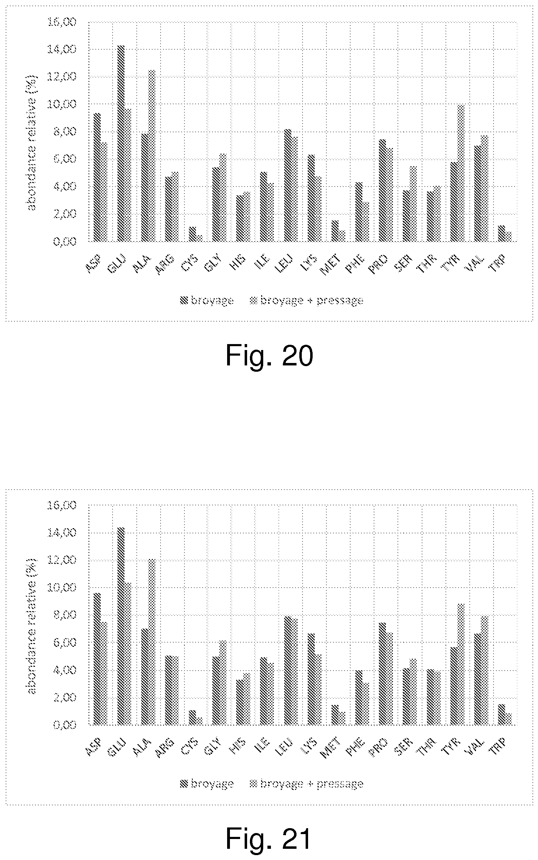

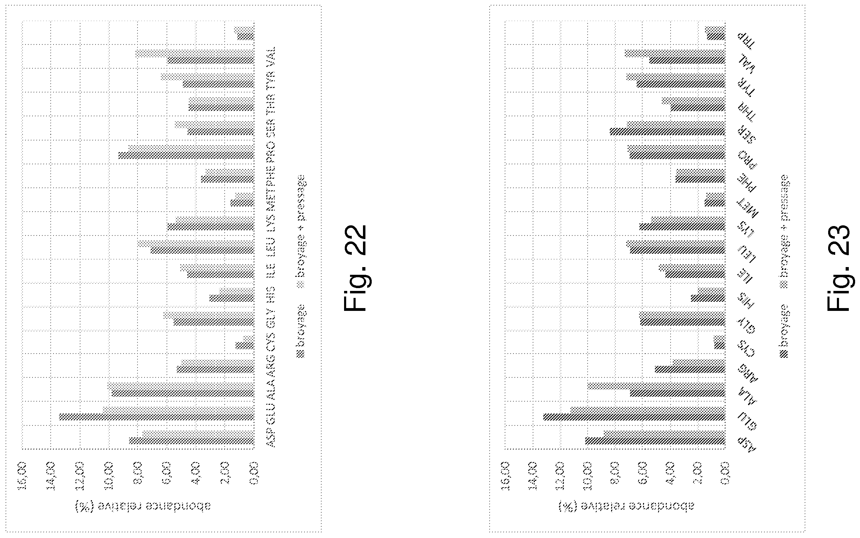

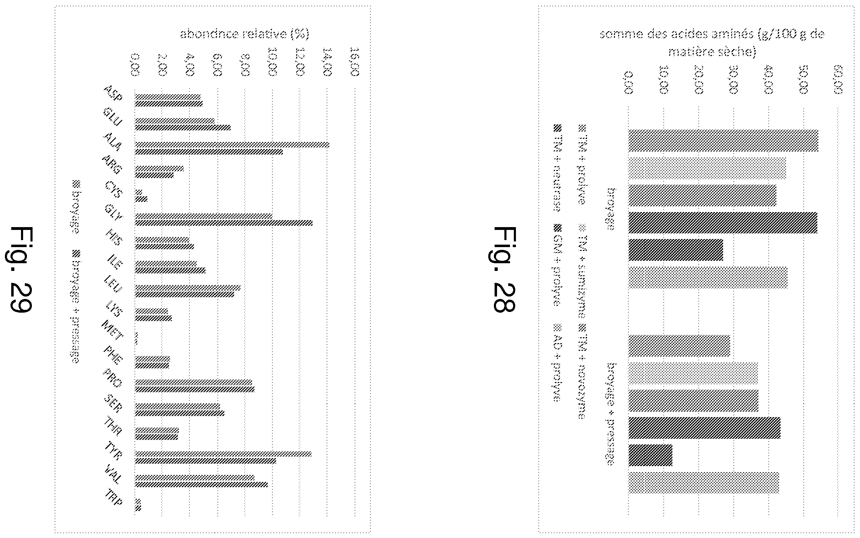

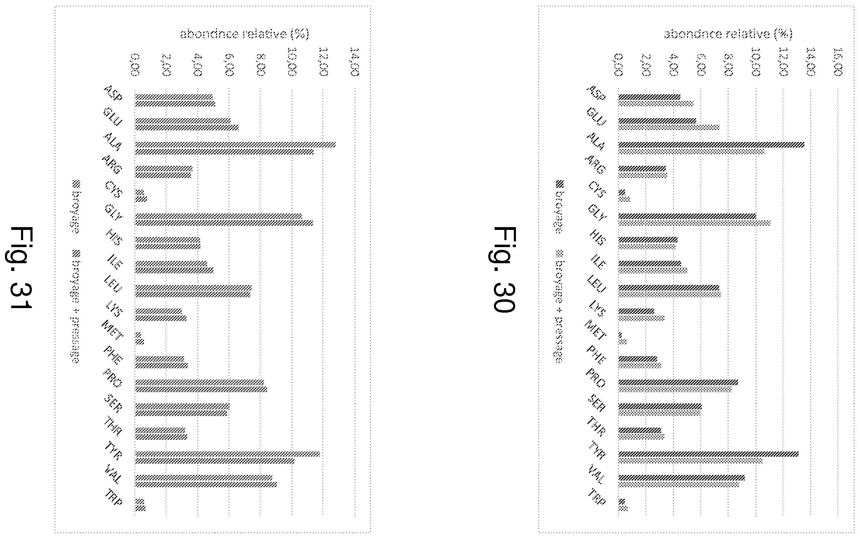

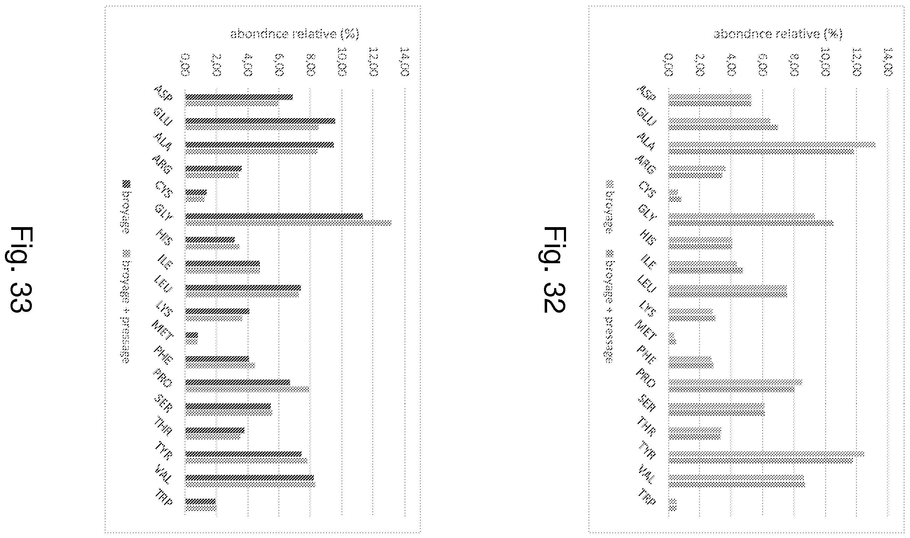

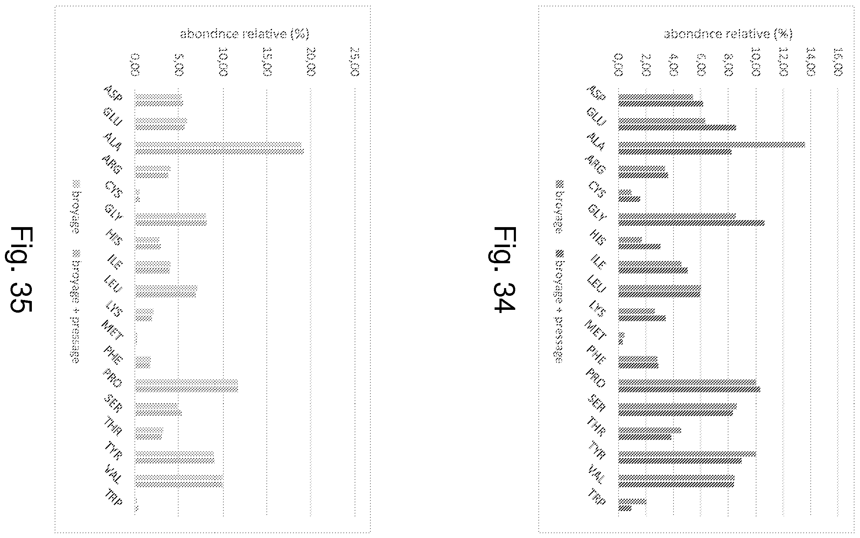

L'utilisation de l'étape de pressage permet une meilleure extraction des acides aminés davantage présents au niveau de la cuticule, tels que l'alanine et la tyrosine et, dans une moindre mesure, la valine, la sérine et la glycine, et ce quel que soit l'enzyme ou l'insecte étudiés (Figures 18 - 24). Ces résultats sont également à mettre en rapport avec ceux concernant les acides aminés présents au niveau de la chitine purifiée enzymatiquement (Figure 29-35).

Remarque : si l'abondance relative de certains acides aminés, dont l'acide aspartique et le glutamate, notamment, diminue, leurs quantités restent identiques, voire, augmentent légèrement dans certains cas, puisque la quantité totale des acides aminés extraits par le procédé avec une étape de pressage augmente (cf. taux de protéines dans l'hydrolysat)

**III. Chitine**

a) Cendres

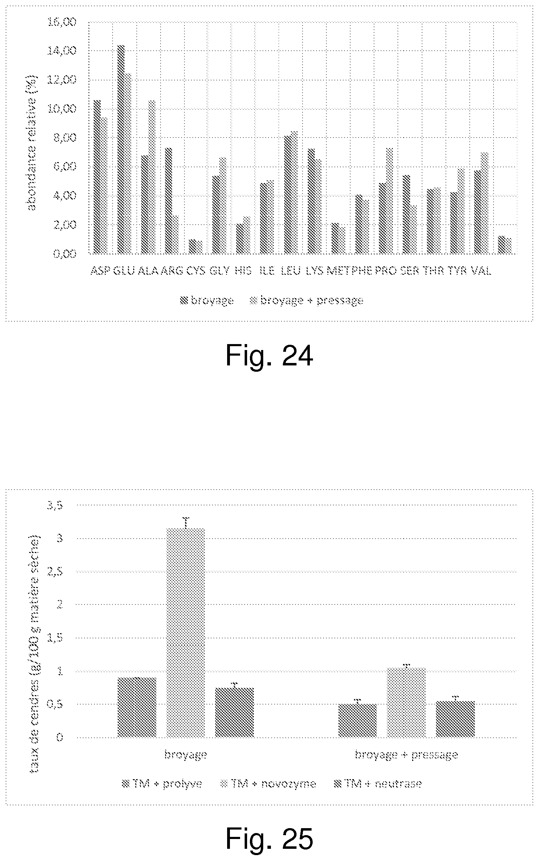

Le taux de cendres dans la chitine est également affecté par l'étape de pressage, bien que dans une moindre mesure que pour l'hydrolysat. On observe ainsi une dimuniton allant de 25% à 28,6% en fonction de l'enzyme utilisée (Figure 25).

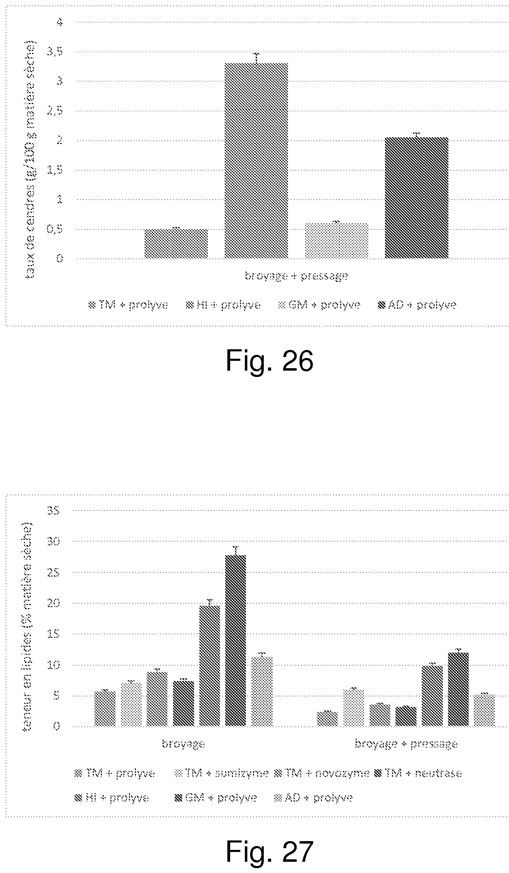

Le taux de cendres dans la chitine (obtenue par le procédé broyage + pressage) est extrêmement bas, et ce quel que soit l'insecte (Figure 26) étudié. Ainsi, dans le cas de *T. molitor,* le taux maximum de cendres est de 1,05 g/100 g de matière sèche, et même pour les insectes naturellement riches en minéraux il reste inférieur à 3,5 g/100 g de matière sèche.

b) Teneur en lipides dans la chitine

Bien que dans une mesure légèrement moindre que pour l'hydrolysat, la teneur en lipides dans la chitine diminue également lorsque l'étape de pressage est ajoutée au procédé (Figure 27). L'efficacité dépend essentiellement de l'enzyme, de 15% pour la sumizyme et jusqu'à 60% pour la novozyme. Quel que soit l'insecte, cette diminution se situe entre 50% dans le cas de *H. illucens* et 58% dans le cas de *T. molitor,* lorsque la réaction est réalisée avec la prolyve.

Pour l'ensemble des insectes, le taux de lipides est inférieur à 12%, et pour les insectes non volants il est même ≤6%.

c) Taux et abondance relative d'acides aminés dans la chitine

L'étape de pressage permet l'élimination d'une plus grande partie des protéines accrochées à la chitine. La mesure directe du taux de protéines étant rendue difficilie étant donné la fonction amide présentes au niveau de la structure même de la chitine, nous avons approché ce taux de protéines par la somme des acides aminés (Figure 28). Ainsi, on peut constater que la somme des acides aminés diminue, de 5 à 54%, lorsque l'étape de pressage est ajoutée au procédé et ce quel que soit l'enzyme ou l'insecte étudié.

L'abondance relative des acides aminés est faiblement affectée par l'étape de pressage, quelques acides aminés néanmoins, dont l'alanine, semblent mieux extraits lorsque l'étape de pressage est réalisée (Figures 29 à 35). Néanmoins nous pouvons constater que pour l'ensemble des insectes, l'alanine, la tyrosine et la proline, ainsi que, dans une moindre mesure, la valine, la glycine, la leucine et la sérine sont des acides aminés principalement attachés à la chitine, leur taux est compris en moyenne entre 23 - 40% de l'ensemble des acides aminés, et c'est également parmi ces acides aminés que l'on note les diminutions les plus importantes au niveau de la chitine et les augmentations les plus importantes (on arrive à 7-30% maximum) au niveau de l'hydrolysat (Figures 18-24) lorsque l'on rajoute une étape de presse dans le procédé.

#### d) Pureté gravimétrique

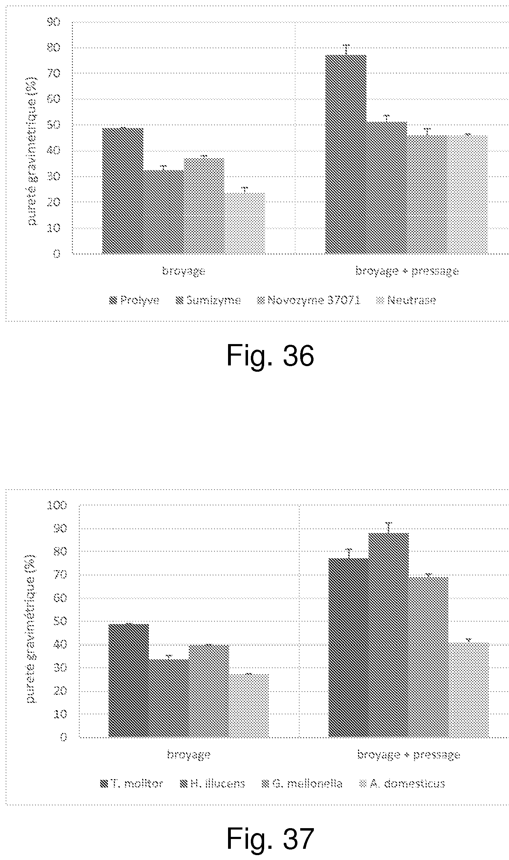

Quel que soit l'enzyme (Figure 36) ou l'insecte (Figure 37) étudiés, la pureté gravimétrique est améliorée par l'utilisation de l'étape de pressage. Elle passe ainsi de 48,7% à 77,1% dans le cas de *T. molitor* avec la prolyve et de 33,7% à 87,9% dans le cas de *H. illucens* avec prolyve.

#### e) Pureté colorimétrique

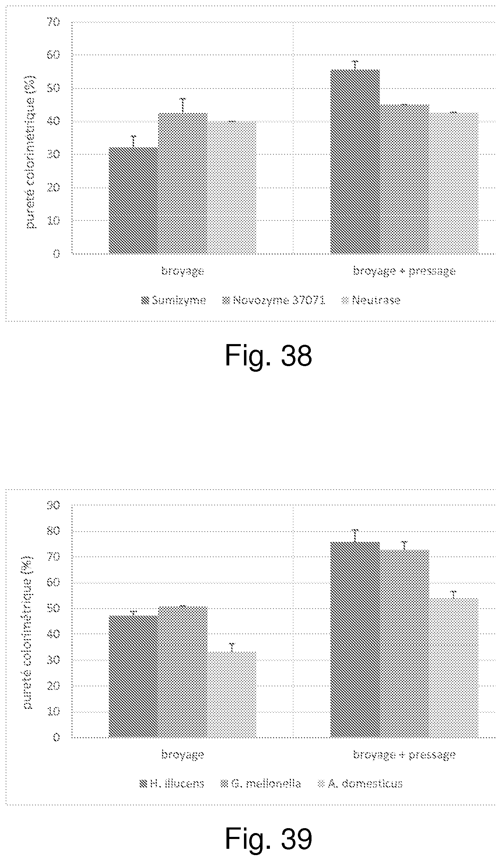

Une amélioration, certes, moins nette par rapport à la pureté gravimétrique, de la pureté colorimétrique est également observée lorsque l'étape de pressage fait partie du procédé, et ce quel que soit l'enzyme ou l'insecte étudié (Figures 38 et 39).

#### f) Pureté par différence

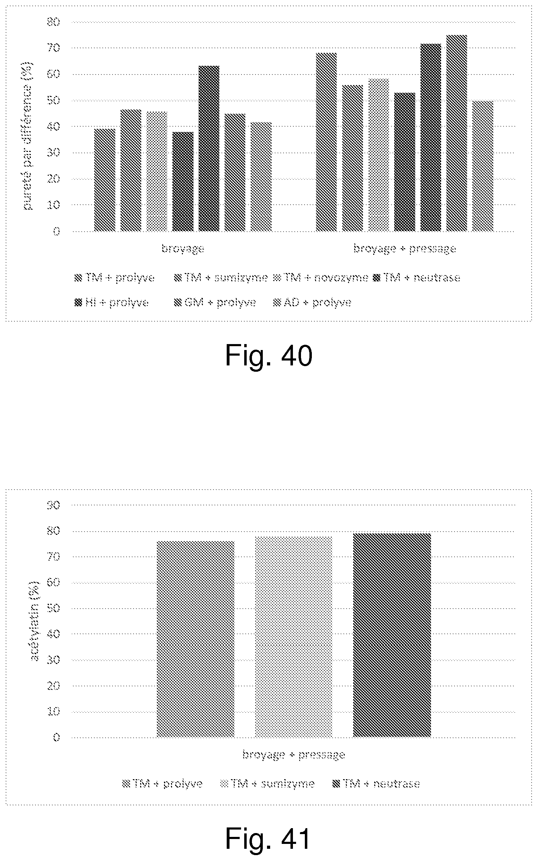

Du fait de la diminution du taux de lipides, de cendres et d'acides aminés dans la chitine obtenue après le procédé avec pressage, la pureté par différence de cette chitine, augemente de manière significative, de 13% dans le cas de *H. illucens* et jusqu'à 74% dans celui de *T. molitor* avec prolyve, et ce quel que soit l'insecte ou l'enzyme étudiés (Figure 40).

#### g) Degré d'acétylation de la chitine

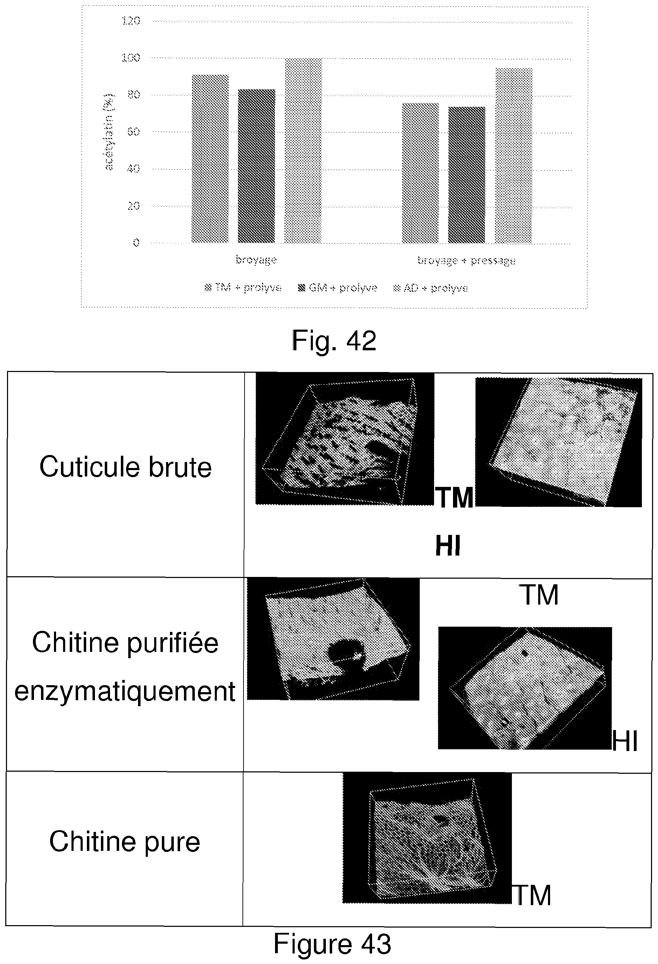

Le degré d'acétylation est significativement affecté par l'ajout de l'étape de pressage dans le procédé (Figures 41 et 42). En effet, l'élimination des lipides et acides aminés de l'hémolymphe permet une plus grande accessiilité des enzymes à la surface de la cuticule, ce qui contribue à l'amélioration du clivage des liaisons peptidiques des protéines liées à la chitine, mais également, par promiscuité catalytique, clivage des liaiasons amides de la chtine. Ainsi le degré d'acétylation de la chitine de *T. molitor* issue du procédé est compris entre 76 et 79%, alors qu'en l'absence de l'étape de pressage, il est à 91% et même l'hydrolyse chimique, réalisée dans des conditions drastiques de température et réactifs conduit à un degré d'acétylation de 85%.

Pour les autres insectes considérés, notamment *G. melonella* et *A. domesticus,* le degré d'acétylation est également affecté par le procédé, ainsi il passe de 83 à 74% dans un cas et de 100 à 95% dans l'autre, respectivement.

La solubilité et la procèssabilité de la chitine étant tout particulièrement liées à son degré d'acétylation, cette hydrolyse concomittante de la liaison amide des unités *N*-acétyl-glucosamine de la chitine, est un effet positif inattendu de l'hydrolyse enzymatique réalisée dans ces conditions.

#### h) Abondances atomique et fonctionnelle à la surface

La répartition des atomes à la surface de la chitine au fur et à mesure de la purification montre une progression du taux relatif d'oxygène et une diminution du taux relatif de carbone (Tableau 4).

De même, les liaisons des atomes de carbone à la surface tendent à passer de liaisons majoritairement de type hydrocarbone (C-H, C-C), vers des liaisons de type alcool (C-O) ou carbonyle (C=O). Les liaisons de type amide (N-C=O) diminuent également.

Ce qui, néanmoins, semble le plus représentatif, c'est le ratio liaison alcool/liaison hydrocarbone (C-O/C-H), dans le cadre de la chitine purifiée enzymatiquement ce ratio est compris entre 0,31 et 0,56, mais plus spécifiquement entre 0,39 et 0,41.

Dans ce Tableau 4, par :

- « cuticule brute », on vise la chitine de la cuticule à l'état natif, analysée directement après arrachage à l'insecte par dissection ;
- « chitine purifiée enzymatiquement (novozyme) », on vise une chitine issue d'un procédé comportant un broyage + pressage et une hydrolyse enzymatique en présence de l'enzyme Novozyme 37071 ;
- « chitine purifiée enzymatiquement (prolyve) », on vise une chitine issue d'un procédé comportant un broyage + pressage et une hydrolyse enzymatique en présence de l'enzyme Prolyve NP ;
- « chitine purifiée enzymatiquement », on vise une chitine issue d'un procédé comportant un broyage + pressage et une hydrolyse enzymatique en présence de l'enzyme prolyve ;
- « chitine pure », on vise une chitine obtenue par purification chimique, identique à celle utilisée pour la détermination de la quantité de la chitine dans l'insecte

| | | atomes | | types de liaisons | | | | ratio |
|---|---|---|---|---|---|---|---|---|
| | | C | O | C-H | C-O | C=O | N-C=O | (C-O)/ (C-H) |
| TM | cuticule brute | 78,85 | 16,23 | 68,88 | 20,16 | 6,87 | 3,53 | 0,29 |
| TM | chitine purifiée enzymatiquement (novozyme) | 75,71 | 18,50 | 62,88 | 24,21 | 9,93 | 2,98 | 0,39 |
| TM | chitine purifiée enzymatiquement (prolyve) | 75,64 | 19,40 | 57,54 | 29,57 | 10,67 | 2,22 | 0,51 |
| TM | chitine pure | 64,24 | 30,31 | 39,5 | 42,36 | 16,21 | 1,92 | 1,07 |
| AD | chitine purifiée enzymatiquement | 78,49 | 16,37 | 68,16 | 20,84 | 8,48 | 2,32 | 0,31 |
| AD | chitine pure | 67,27 | 27,30 | 42,32 | 40,46 | 13,59 | 3,63 | 0,96 |
| GM | chitine purifiée enzymatiquement | 76,24 | 19,29 | 63,15 | 25,15 | 8,9 | 2,81 | 0,40 |
| GM | chitine pure | 64,93 | 28,87 | 34,1 | 45,39 | 18,07 | 2,44 | 1,33 |
| HI | cuticule brute | 86,09 | 12,69 | 83,35 | 8,41 | 2,55 | 5,68 | 0,10 |
| HI | chitine purifiée enzymatiquement | 74,46 | 21,10 | 61,49 | 25,07 | 7,95 | 5,49 | 0,41 |
| HI | chitine pure | 73,10 | 22,49 | 53 | 30,3 | 11,16 | 4,11 | 0,57 |

**Tableau 4.** Répartition de certains atomes et des liaisons à la surface de la chitine

i) Représentation de la cuticule par microscopie par fluorescence bi-photon

La purification partielle de la chitine par voie enzymatique selon le procédé selon l'invention permet le maintien de la souplesse de l'ensemble par la préservation de « remplissage » protéique des structures chitiniques, comparé à une purification chimique, tout en enlevant la surcharge protéique à l'extérieur de la structure (voir Figure 43).

La terminologie employée sur la Figure 43 est la même que celle définie au point III h) ci-avant.

j) Degré de cristallinité

Quels que soient l'insecte ou l'enzyme étudié, le degré de cristallinité, i.e. le rapport des aires cristallines et amorphes, de la chitine obtenue est compris entre 0,42 et 0,61.

Généralement, on parle dans la littérature de l'index de cristallinité, i.e. rapport des hauteurs des pics (et non des aires). Cette mesure comporte un risque d'erreur important. Néanmoins, et à des fins de comparaison, l'index de cristallinité des échantillons a également été mesuré, et il se situe entre 88 et 95% pour l'ensemble des insectes, et est même supérieur à 90% pour les coléoptères et les lépidoptères

**Exemple 6** : **Procédé selon l'invention avec récupération de la fraction protéique issue du jus de presse**

**I. Matériel et méthodes**

a) Matériel

**Insecte**

L'insecte suivant a été étudié :

- un coléoptère : *Tenebrio molitor* (*T. molitor* ou TM).

**Enzyme**

La Prolyve a été mise en oeuvre au cours de l'hydrolyse.

| enzyme | Prolyve |
|---|---|
| Activité enzymatique | 3789,52 |

| voulue | |
|---|---|
| Activité enzymatique / g | 3789,52 |
| m (g) | 1,00 |

b) Procédés de production

***Procédé de production avec broyage suivi d'un pressage (désigné « broyage + pressage » dans les figures)***

Plusieurs batchs ont été transformés de façon suivante : 600 g de larves de T. *molitor,* frais sont introduite dans une enceinte où ils sont abattus à la vapeur d'eau (115 °C, 5 minutes). Les insectes sont ensuite introduits dans un appareil de mixage et 75 mL d'eau pour 100 g d'insectes sont ajoutés, l'ensemble est ensuite mixé et pressé (presse bi-vis, ou filtre presse, ou autre système de pressage).
Le gâteau de presse est ensuite séché, 70 °C durant la nuit. Le jus de presse, en revanche, est centrifugé pendant 30 minutes avec une force de 3000 g et la partie solide est récupérée - protéines insolubles de l'hémolymphe.
125 g de gâteau de presse et 125 g (poids humide, 30% poids sec environ) de protéines insolubles de l'hémolymphe sont ensuite introduits dans un ballon tricol muni d'un réfrigérant et d'un agitateur magnétique, 1250 mL d'eau sont ajoutés ainsi qu'une enzyme protéolytique d'une activité équivalente à 9500 SAPU. La réaction est ensuite portée à 45 °C pendant 4 heures. La température est ensuite portée à 90 °C pendant 15 minutes, le mélange réactionnel est enfin filtré (0,40 - 0,45 $\mu$m). Le résidu est séché pendant 24 heures à 70 °C : il s'agit donc de la chitine obtenue par voie enzymatique de purification ; le filtrat est congelé et est lyophilisé : il s'agit donc de l'hydrolysat.

c) Analyses

La mesure du taux de cendres, la mesure de la teneur en lipides, l'abondance relative en acides aminés et la mesure de la taille des protéines ont été réalisées comme dans l'exemple 5.

## II. Caractérisation des produits obtenus

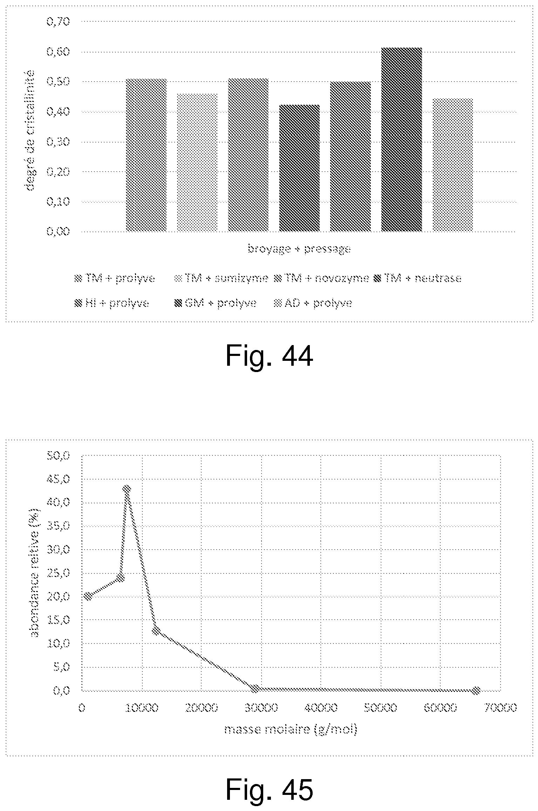

Les produits obtenus, la chitine et l'hydrolysat, ont été analysés et caractérisés (Tableau 5, Figure 45).

**Tableau 5** : Caractérisation

|  | hydrolysat | Chitine |
|---|---|---|
| Cendres | 4.1 | 0.5 |
| Lipides | 12.85 | 3.7 |
| digestibilité | 95 | n.a. |
| Acides aminés (abondance relative (%)) | | |
| ASP | 9.7 | 5.4 |
| GLU | 12.8 | 6.7 |
| ALA | 9.0 | 13.7 |
| ARG | 2.5 | 3.6 |
| CYS | 1.1 | 0.5 |
| GLY | 5.9 | 9.4 |
| HIS | 3.3 | 3.9 |
| ILE | 5.4 | 4.8 |
| LEU | 8.5 | 8.0 |
| LYS | 6.5 | 2.9 |
| MET | 1.5 | 0.4 |
| PHE | 4.0 | 2.6 |
| PRO | 6.9 | 8.3 |
| SER | 4.4 | 5.8 |
| THR | 4.9 | 3.5 |
| TYR | 4.7 | 11.3 |
| VAL | 7.7 | 9.0 |
| TRP | 1.3 | 0.6 |
| n.a. non applicable | | |

## Revendications

1. Hydrolysat préparé à partir d'insectes comportant au moins 40% de protéines en poids sur le poids total de matière sèche, un taux de cendres inférieur à 3% en poids sur le poids total de matière sèche, et un taux de protéines hydrosolubles ayant une taille supérieure à 12400 g/mol inférieur à 50%.

2. Procédé de production d'au moins un produit d'intérêt à partir d'insectes, comportant les étapes suivantes :

       (i) le broyage des cuticules d'insectes,
       (ii) le pressage des cuticules d'insectes, puis,
       (iii) l'hydrolyse enzymatique des cuticules d'insectes par une enzyme protéolytique.

3. Procédé selon la revendication 2, comportant une étape d'abattage des insectes préalable à l'étape de broyage.

4. Procédé selon la revendication 2 ou 3, comportant en outre une étape de traitement des cuticules des insectes par un agent oxydant préalablement à l'hydrolyse enzymatique.

**5.** Procédé selon l'une quelconque des revendications 2 à 4, dans lequel le ou les insecte(s) est/sont choisi(s) parmi le groupe constitué par les coléoptères, les lépidoptères, les orthoptères et les diptères.

**6.** Procédé selon l'une quelconque des revendications 2 à 5, dans lequel la protéase est choisie parmi le groupe constitué par les aminopeptidases, les métallocarboxypeptidases, les endopeptidases sérine, les endopeptidases cystéine, les endopeptidases aspartiques, les métalloendopeptidases.

**7.** Procédé selon l'une quelconque des revendications 2 à 6, dans lequel le produit d'intérêt est la chitine et/ou le chitosan.

**8.** Procédé selon l'une quelconque des revendications 2 à 6, dans lequel le produit d'intérêt est un hydrolysat.

**9.** Procédé de production de chitine à partir de cuticules d'insectes, comportant les étapes suivantes :

    a) l'abattage des insectes,
    b) le broyage des insectes,
    c) le pressage des insectes,
    d) l'hydrolyse enzymatique des cuticules d'insectes par une enzyme protéolytique,
    e) la récupération de la chitine,
    les cuticules d'insectes pouvant optionnellement être traitées avec un agent oxydant avant l'étape d).

**10.** Chitine susceptible d'être obtenue par un procédé selon l'une quelconque des revendications 2 à 7, 9.

**11.** Procédé de production d'un hydrolysat à partir d'insectes, comportant les étapes suivantes :

    a) l'abattage des insectes,
    b) le broyage des insectes,
    c) le pressage des insectes,
    d) l'hydrolyse enzymatique des cuticules d'insectes par une enzyme protéolytique,
    e) la récupération de l'hydrolysat,
    les cuticules d'insectes pouvant optionnellement être traitées avec un agent oxydant avant l'étape d).

**12.** Hydrolysat susceptible d'être obtenu par un procédé selon l'une quelconque des revendications 2 à 6, 8, 11.

**13.** Procédé de production de chitosan à partir de cuticules d'insectes, comportant les étapes suivantes :

    a) l'abattage des insectes,
    b) le broyage des insectes,
    c) le pressage des insectes,
    d) l'hydrolyse enzymatique des cuticules d'insectes par une protéase,
    e) la récupération de la chitine,
    f) la désacétylation de la chitine récupérée,
    g) la récupération du chitosan,
    les cuticules d'insectes pouvant optionnellement être traitées avec un agent oxydant avant l'étape d).

**14.** Chitine comportant un taux d'acides aminés inférieur ou égal à 45% en poids sur le poids total de matière sèche, un taux de cendres inférieur ou égal à 2,5% en poids sur le poids total de matière sèche, une pureté par différence supérieure ou égale à 45%, un degré d'acétylation supérieur ou égal à 70% et un degré de cristallinité supérieur ou égal à 0,42.

**Patentansprüche**

**1.** Hydrolysat, hergestellt ausgehend von Insekten, welches umfasst: mindestens 40 Gewichtsprozent Proteine, bezogen auf das Gesamtgewicht an Trockensubstanz, einen Aschenanteil von unter 3 Gewichtsprozent, bezogen auf das Gesamtgewicht an Trockensubstanz, und einen Anteil von unter 50% an wasserlöslichen Proteinen, die eine Größe von über 12400 g/mol aufweisen.

**2.** Verfahren zur Herstellung mindestens eines Produktes, das von Interesse ist, ausgehend von Insekten, welches die folgenden Schritte umfasst:

(i) Zerkleinern der Insekten-Cuticulae,
(ii) Pressen der Insekten-Cuticulae, anschließend
(iii) enzymatische Hydrolyse der Insekten-Cuticulae durch ein proteolytisches Enzym.

**3.** Verfahren nach Anspruch 2, welches einen Schritt der Tötung der Insekten umfasst, der dem Zerkleinerungsschritt vorangeht.

**4.** Verfahren nach Anspruch 2 oder 3, welches vor der enzymatischen Hydrolyse zudem einen Schritt der Behandlung der Cuticulae der Insekten mit einem Oxidationsmittel umfasst.

**5.** Verfahren nach einem der Ansprüche 2 bis 4, in welchem das oder die Insekt(en) ausgewählt ist/sind aus der Gruppe bestehend aus Coleoptera, Lepidoptera, Orthoptera und Diptera.

**6.** Verfahren nach einem der Ansprüche 2 bis 5, in welchem die Protease ausgewählt ist aus der Gruppe bestehend aus Aminopeptidasen, Metallocarboxypeptidasen, Serin-Endopeptidasen, Cystein-Endopeptidasen, Aspartat-Endopeptidasen und Metalloendopeptidasen.

**7.** Verfahren nach einem der Ansprüche 2 bis 6, in welchem das Produkt, das von Interesse ist, Chitin und/oder Chitosan ist.

**8.** Verfahren nach einem der Ansprüche 2 bis 6, in welchem das Produkt, das von Interesse ist, ein Hydrolysat ist.

**9.** Verfahren zur Herstellung von Chitin ausgehend von Insekten-Cuticulae, welches die folgenden Schritte umfasst:

a) Tötung der Insekten,
b) Zerkleinerung der Insekten,
c) Pressen der Insekten,
d) enzymatische Hydrolyse der Insekten-Cuticulae durch ein proteolytisches Enzym,
e) Gewinnung des Chitins,
wobei die Insekten-Cuticulae vor dem Schritt d) optional mit einem Oxidationsmittel behandelt werden können.

**10.** Chitin, welches geeignet ist, durch ein Verfahren nach einem der Ansprüche 2 bis 7 oder 9 erhalten zu sein.

**11.** Verfahren zur Herstellung eines Hydrolysats, ausgehend von Insekten, welches die folgenden Schritte umfasst:

a) Tötung der Insekten,
b) Zerkleinerung der Insekten,
c) Pressen der Insekten,
d) enzymatische Hydrolyse der Insekten-Cuticulae durch ein proteolytisches Enzym,
e) Gewinnung des Hydrolysats,
wobei die Insekten-Cuticulae vor dem Schritt d) optional mit einem Oxidationsmittel behandelt werden können.

**12.** Hydrolysat, das geeignet ist, durch ein Verfahren nach einem der Ansprüche 2 bis 6, 8 oder 11 erhalten zu sein.

**13.** Verfahren zur Herstellung von Chitosan, ausgehend von Insekten-Cuticulae, welches die folgenden Schritte umfasst:

a) Tötung der Insekten,
b) Zerkleinerung der Insekten,
c) Pressen der Insekten,
d) enzymatische Hydrolyse der Insekten-Cuticulae durch eine Protease,
e) Gewinnung des Chitins,
f) Desacetylierung des gewonnenen Chitins,
g) Gewinnung des Chitosans,
wobei die Insekten-Cuticulae vor dem Schritt d) optional mit einem Oxidationsmittel behandelt werden können.

**14.** Chitin, welches umfasst: einen Aminosäuren-Anteil von kleiner oder gleich 45 Gewichtsprozent, bezogen auf das Gesamtgewicht an Trockensubstanz, einen Aschenanteil von kleiner oder gleich 2,5 Gewichtsprozent, bezogen auf das Gesamtgewicht an Trockensubstanz, eine Reinheit mittels Differenz von größer oder gleich 45%, einen Acetylierungsgrad von größer oder gleich 70% und einen Kristallinitätsgrad von größer oder gleich 0,42.

**Claims**

**1.** Hydrolysate prepared from insects comprising at least 40% by weight of proteins based on the total weight of dry matter, an ash content of less than 3% by weight based on the total weight of dry matter, and a content of watersoluble proteins having a size larger than 12,400 g/mol of less than 50%.

**2.** Method for the production of at least one product of interest from insects, comprising the following steps:

(i) grinding the insect cuticles,
(ii) pressing the insect cuticles, and then
(iii) enzymatic hydrolysis of the insect cuticles with a proteolytic enzyme.

**3.** Method according to claim 2, comprising a step of killing the insects prior to the grinding step.

**4.** Method according to claim 2 or 3, further comprising a step of treatment of the insect cuticles with an oxidizing agent prior to enzymatic hydrolysis.

**5.** Method according to any one of claims 2 to 4, in which the insect or insects is/are selected from the group constituted by the Coleoptera, the Lepidoptera, the Orthoptera and the Diptera.

**6.** Method according to any one of claims 2 to 5, in which the protease is selected from the group constituted by aminopeptidases, metallocarboxypeptidases, serine endopeptidases, cysteine endopeptidases, aspartic endopeptidases, metalloendopeptidases.

**7.** Method according to any one of claims 2 to 6, in which the product of interest is chitin and/or chitosan.

**8.** Method according to any one of claims 2 to 6, in which the product of interest is a hydrolysate.

**9.** Method for the production of chitin from insect cuticles, comprising the following steps:

a) killing the insects,
b) grinding the insects,
c) pressing the insects,
d) enzymatic hydrolysis of the insect cuticles with a proteolytic enzyme,
e) recovery of the chitin,
the insect cuticles optionally being able to be treated with an oxidizing agent before step d).

**10.** Chitin obtainable by a method according to any one of claims 2 to 7, and 9.

**11.** Method for the production of a hydrolysate from insects, comprising the following steps:

a) killing the insects,
b) grinding the insects,
c) pressing the insects,
d) enzymatic hydrolysis of the insect cuticles with a proteolytic enzyme,
e) recovery of the hydrolysate,
the insect cuticles optionally being able to be treated with an oxidizing agent before step d).

**12.** Hydrolysate obtainable by a method according to any one of claims 2 to 6, 8, and 11.

**13.** Method for the production of chitosan from insect cuticles, comprising the following steps:

a) killing the insects,
b) grinding the insects,
c) pressing the insects,
d) enzymatic hydrolysis of the insect cuticles with a protease,
e) recovery of the chitin,
f) deacetylation of the chitin recovered,
g) recovery of the chitosan,
the insect cuticles optionally being able to be treated with an oxidizing agent before step d).

14. Chitin comprising an amino acid content less than or equal to 45% by weight based on the total weight of dry matter, an ash content less than or equal to 2.5% by weight based on the total weight of dry matter, a purity by difference greater than or equal to 45%, a degree of acetylation greater than or equal to 70% and a degree of crystallinity greater than or equal to 0.42.

Fig. 1

EP 3 240 902 B1

Fig.2

pureté de la chitine (%)

Broyage 1 | Broyage 1 + pressage | Broyage 1 + pressage + broyage 2 | Pressage

(%)

taux des lipides (%) dans l'intermédiaire dont la chitine a été extraite

Fig. 3

▒ pureté de la chitine
● tx des lipides dans l'hydrolysat
⬧ tx des lipides dans la chitine

Fig. 4

tx de lipides dans le gâteau de presse
tx de lipides dans le jus de presse

poids moléculaire (Da)

Fig. 5    ---○--- jus de presse    ▲ hydrolysat

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 30

Fig. 31

Fig. 32

Fig. 33

Fig. 34

Fig. 35

Fig. 36

Fig. 37

Fig. 38

Fig. 39

Fig. 40

Fig. 41

Fig. 42

| Cuticule brute | |
| Chitine purifiée enzymatiquement | |
| Chitine pure | |

Figure 43

Fig. 44

Fig. 45

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **LOELOVICH.** *M. Res. Rev.: J. Chem.,* 2014, vol. 3, 7-14
- **SIMIONATTO GUINESI, L. ; GOMES CAVALHEIRO, E. T.** *Thermochimica Acta,* 2006, vol. 444, 128-133
- **HEUX, L. ; BRUGNEROTTO, J. ; DESBRIÈRES, J. ; VERSALI, M.-F. ; RINAUDO, M.** *Biomacromolecules,* 2000, vol. 1, 746-751